# FASCICULE DE BREVET EUROPEEN

(11) **EP 2 051 985 B1**
(45) Date de publication et mention de la délivrance du brevet: **16.04.2014**
(21) Numéro de dépôt: 07823379.8
(22) Date de dépôt: 31.07.2007
(51) Int. Cl.: C07D 513/04, C07D 277/38, C07D 231/44, A61K 31/429, A61K 31/4162, A61P 35/00

(54) **DERIVES DE PYRAZOLO[4,3-D]THIAZOLE, LEUR PREPARATION ET LEUR APPLICATION EN THERAPEUTIQUE**
PYRAZOLO[4,3-D]THIAZOL-DERIVATE, ZUBEREITUNG UND THERAPEUTISCHE ANWENDUNG
PYRAZOLO[4,3-D]THIAZOLE DERIVATIVES, AND PREPARATION AND THERAPUETIC APPLICATION THEREOF

(30) Priorité: 03.08.2006 FR 0607128
(43) Date de publication de la demande: 29.04.2009
(73) Titulaire: SANOFI, 75008 Paris (FR)
(72) Inventeur: DOERFLINGER, Gilles, 75013 Paris (FR); CARRY, Jean-Christophe, 75013 Paris (FR)
(74) Mandataire: Jacobson, Claude
(86) Numéro de dépôt international: PCT/FR2007/001326
(87) Numéro de publication internationale: WO 2008/015340

(56) Documents cités:
- WO-A-2005/068473
- WO-A-2005/074922
- US-A1- 2005 026 984

## Description

La présente invention se rapporte à des dérivés de pyrazolo[4,3-d]thiazole, les compositions les contenant et leur application en thérapeutique, notamment comme anticancéreux. L'invention se rapporte aussi au procédé de préparation de ces composés ainsi qu'à certains des produits intermédiaires.

### [Domaine technique]

A ce jour, la plupart des composés commerciaux utilisés en chimiothérapie posent des problèmes importants d'effets secondaires et de tolérance par les patients. La recherche de nouveaux agents anticancéreux s'est orientée ces dernières années vers des thérapies ciblant des enzymes ou d'autres biomolécules exprimées et/ou activées majoritairement dans les cellules cancéreuses. Une classe importantes d'enzymes ayant fait l'objet de nombreuses études est la famille des protéines kinases.

### [L'art antérieur]

La demande EP 1510516 décrit des composés inhibiteurs de kinases JNK, pouvant être utilisés dans des maladies neurodégénératives (Alzheimer, Parkinson). La demande WO 2004/013146 décrit des composés inhibiteurs de kinases, pouvant être utilisés dans le traitement des cancers. La demande US 2005/0176786 décrit des composés inhibiteurs de kinases tyrosine, pouvant être utilisés dans le traitement des cancers. La demande US 2005/0187209 décrit des composés inhibiteurs de kinases, notamment Aurora 2, pouvant être utilisés dans le traitement des cancers. La demande WO 2005/095420 décrit des composés inhibiteurs de certaines kinases, pouvant être utilisés dans le traitement du psoriasis ou du cancer du cerveau.

Les demandes WO 2005/074922 et US 2005/0026984 décrivent des composés du type thiéno [2,3-c]pyrazoles. La demande WO 2005/068473 décrit pour X=S, des composés du type thiazolo [2,4-d]pyrazoles.

La demande WO 2007/059341 décrit des composés ayant pour formule :

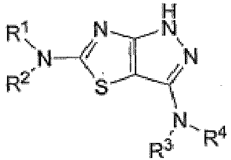

Aucun de ces documents ne décrit ni ne suggère les composés du type thiazolo [4,3-d]pyrazoles de la présente invention.

### [Description de l'invention]

L'invention est relative à un composé de formule (I) : 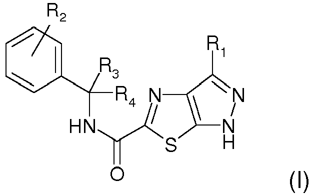 tel que défini à l'une des revendications 1 à 5. Ce composé peut aussi exister sous forme d'hydrate ou de solvat et/ou de sel d'addition.

L'invention est aussi relative à un médicament comprenant le composé précédent ainsi qu'à une composition pharmaceutique comprenant ledit composé ainsi qu'au moins un excipient pharmaceutiquement acceptable.

L'invention est aussi relative à l'utilisation du composé précédent pour la préparation d'un médicament destiné au traitement ou à la prévention du cancer.

L'invention est relative aussi au procédé d'obtention du composé ainsi qu'à certains des intermédiaires.

### [Description détaillée de l'invention]

### Définitions utilisées

Dans le cadre de la présente invention, et sauf mention différente dans le texte, on utilise les définitions suivantes :
- atome d'halogène : un atome de fluor, de chlore, de brome ou d'iode ;
- groupe alkyle : un groupe hydrocarboné aliphatique saturé, linéaire ou ramifiée, comprenant de préférence de 1 à 20 atomes de carbone, avantageusement de 1 à 6, de préférence de 1 à 4 atomes de carbone. On peut notamment citer les groupes suivants : méthyle, éthyle, propyle, butyle, pentyle, hexyle, octyle, nonyle, décyle, dodécyle, hexadécyle, octadécyle, isopropyle, isobutyle, tertiobutyle, 2-éthylhexyle ;
- groupe alcoxy : un groupe -O-alkyle où le groupe alkyle est tel que défini précédemment ;
- groupe cycloalkyle : un groupe alkyle cyclique comprenant de préférence de 3 à 8 atomes de carbone engagés dans la structure cyclique. On peut citer notamment les groupes suivants : cyclopropyle, cyclopentyle, cyclohexyle ;
- groupe aryle : un groupe aromatique cyclique comprenant de préférence de 6 à 14 atomes de carbone. Le groupe aryle est avantageusement mono- ou bicyclique. On peut citer notamment les groupes suivants : phényle, napthyle ;
- groupe hétéroaryle : un groupe aromatique cyclique de 5 à 14 chaînons comprenant comme atomes formant le cycle, un ou plusieurs hétéroatomes choisis parmi O, S ou N. Le groupe hétéroaryle est avantageusement mono- ou bicyclique. De préférence, il comprend entre 2 et 13 atomes de carbone et entre 1 et 8 hétéroatomes. On peut citer les groupes suivants : pyridinyle, pyrimidinyle, pyrazinyle, thiazolyle, imidazolyle, furyle et thiényle ;
- groupe hétérocycloalkyle : un groupe cycloalkyle tel que défini précédemment comprenant en outre comme atomes formant le cycle, un ou plusieurs hétéroatomes choisis parmi N, O ou S. De préférence, il comprend de 1 à 4 hétéroatomes. On peut citer les groupes suivants : azétidinyle, pyrrolidinyle, pipéridinyle, morpholinyle et pipérazinyle. On entend aussi par pipérazinyle les N-alkyle-pipérazinyle de formule 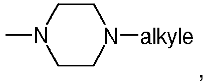 par exemple le N-méthyl-pipérazinyle ;
- C₀: représente une liaison covalente ou un atome d'hydrogène.

On utilise dans la présente demande la nomenclature suivante pour désigner les associations de groupes ; par exemple : « groupe -aryle-(C₀-C₃)alkyle-hétérocycloalkyle » désigne un groupe aryle lié soit à un groupe alkyle lui-même lié à un groupe hétérocycloalkyle soit à un groupe hétérocycloalkyle (dans le cas de C₀). Par exemple, « groupe -aryle-alkyle-cycloalkyle » désigne un groupe aryle lié à un groupe alkyle lui-même lié à un groupe cycloalkyle.

**Selon un premier aspect,** la présente invention a pour objet un composé de formule (I) : 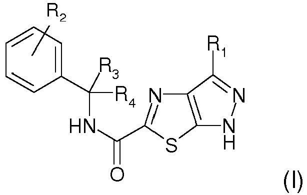 dans laquelle :
(i) R₁ représente un groupe -NHR₅, dans lequel R₅ est sélectionné parmi un atome d'hydrogène ou un groupe -COR₆, où R₆ est choisi parmi un groupe aryle ou hétéroaryle, éventuellement substitué par un atome d'halogène, -(C₁-C₃)alcoxy, -(C₀-C₃)alkyle-hétérocycloalkyle ;
(ii) R₂ représente un atome d'hydrogène ;
(iii) R₃ et R₄ représentent chacun un groupe méthyle ;
dans lequel :
- le groupe hétérocycloalkyle représente un groupe cycloalkyle comprenant de 3 à 8 atomes de carbone, comprenant en outre 1 à 4 hétéroatomes choisis parmi N, O ou S , ledit groupe hétérocycloalkyle étant choisi dans le groupe constitué de : azétidinyle, pyrrolidinyle, pipéridinyle, morpholinyle, pipérazinyle et N-alkyle-pipérazinyle ;
- le groupe alkyle représente un groupe hydrocarboné aliphatique saturé, linéaire ou ramifié comprenant de 1 à 20 atomes de carbone;
- le groupe aryle représente un groupe aromatique cyclique comprenant de 6 à 14 atomes de carbone ;
- le groupe hétéroaryle représente un groupe aromatique cyclique de 5 à 14 chaînons comprenant comme atome formant le cycle, un ou plusieurs hétéroatomes choisis parmi O, S ou N.

Selon un mode de réalisation, le groupe aryle est notamment un phényle et le groupe hétéroaryle est un thiényle ou une pyridine.

Dans le cas où R₆ représente un groupe -aryle-(C₀-C₃)alkyle-hétérocycloalkyle, plus particulièrement dans le cas où l'aryle désigne phényle, thiényle ou pyridine, l'hétérocycloalkyle comprend au moins un atome d'azote sur lequel est relié le groupe aryle ou alkyle (c'est-à-dire le groupe est de la forme -aryle-(C₀-C₃)alkyle-N. L'hétérocycloalkyle est plus particulièrement morpholinyle, pipérazinyle, N-alkyle-pipérazinyle, pyrrolidinyle ou pipéridinyle.

Selon l'invention, on peut également citer plus particulièrement les composés suivants :

| | |
|---|---|
| N-(1-méthyl-1-phényl-éthyl)-(3-amino-1H-pyrazolo[4,3-*d*]thiazole)-5-carboxamide | N-(1-méthyl-1-phényl-éthyl)-3-{[4-(4-méthyl-pipérazin-1-yl)-thiophène-2-carbonyl]-amino}-1H-pyrazolo[4,3-d]thiazole-5-carboxamide |
| 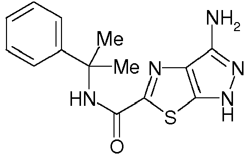 | 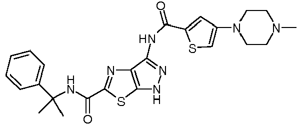 |
| N-(1-méthyl-1-phényl-éthyl)-3-[4-(4-méthyl-pipérazin-1-yl)-benzoylamino]-1H-pyrazolo[4,3-*d*]thiazole-5-carboxamide | N-(1-méthyl-1-phényl-éthyl)-3-{[5-(4-méthyl-pipérazin-1-yl)-thiophène-2-carbonyl]-amino}-1H-pyrazolo[4,3-d]thiazole-5-carboxamide |
| 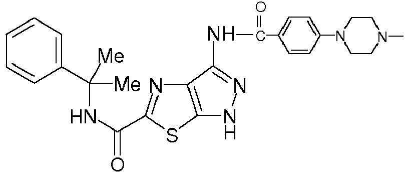 | 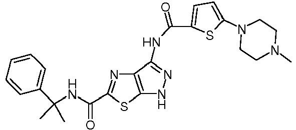 |
| N-(1-méthyl-1-phényl-éthyl)-3-(4-morpholin-4-yl-benzoylamino)-1H-pyrazolo[4,3-*d*]thiazole-5-carboxamide | N-(1-méthyl-1-phényl-éthyl)-3-[(pyridine-2-carbonyl)-amino]-1H-pyrazolo[4,3-d]thiazole-5-carboxamide |
| 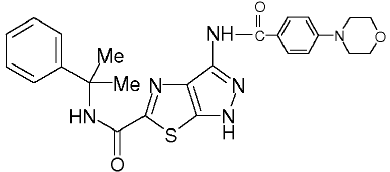 | 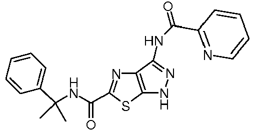 |
| N-(1-méthyl-1-phényl-éthyl)-3-(4-méthoxy-benzoylamino)-1-H-pyrazolo[4,3-*d*]thiazole-5-carboxamide | N-(1-méthyl-1-phényl-éthyl)-3-[(pyridine-3-carbonyl)-amino]-1H-pyrazolo[4,3-d]thiazole-5-carboxamide |
| 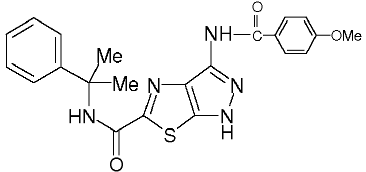 | 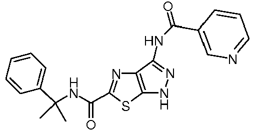 |
| N-(1-méthyl-1-phényl-éthyl)-3-[(thiophène-3-carbonyl)-amino]-1H-pyrazolo[4,3-*d*]thiazole-5-carboxamide | N-(1-méthyl-1-phényl-éthyl)-3-[(pyridine-4-carbonyl)-amino]-1H-pyrazolo[4,3-d]thiazole-5-carboxamide |
| 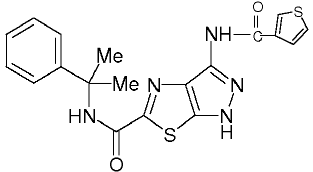 | 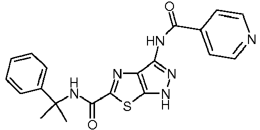 |
| N-(1-méthyl-1-phényl-éthyl)-3-(4-morpholin-4-ylméthyl-benzoylamino)-1H-pyrazolo[4,3-d]thiazole-5-carboxamide | N-(1-méthyl-1-phényl-éthyl)-3-{[2-(4-méthyl-pipérazin-1-yl)-pyridine-4-carbonyl]-amino}-1H-pyrazolo[4,3-d]thiazole-5-carboxamide |
| 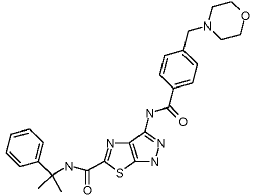 | 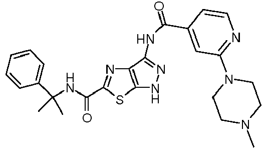 |
| N-(1-méthyl-1-phényl-éthyl)-3-[4-(4-méthyl-piperazin-1-ylméthyl)-benzoylamino]-1H-pyrazolo[4,3-d]thiazole-5-carboxamide | N-(1-méthyl-1-phényl-éthyl)-3-{[6-(4-méthyl-pipérazin-1-yl)-pyridine-2-carbonyl]-amino}-1H-pyrazolo[4,3-d]thiazole-5-carboxamide |
| 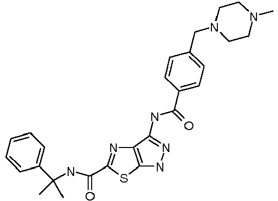 | 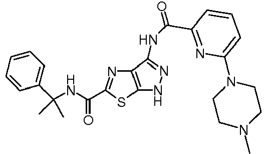 |
| N-(1-méthyl-1-phényl-éthyl)-3-(4-pipéridin-1-ylméthyl-benzoylamino)-1H-pyrazolo[4,3-d]thiazole-5-carboxamide | N-(1-méthyl-1-phényl-éthyl)-3-{[6-(4-méthyl-pipérazin-1-yl)-pyridine-3-carbonyl]-amino}-1H-pyrazolo[4,3-d]thiazole-5-carboxamide |
| 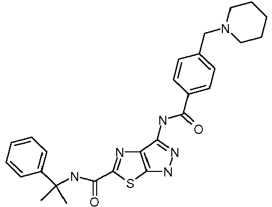 | 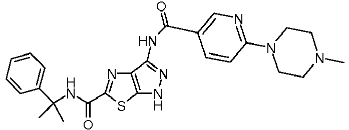 |
| N-(1-méthyl-1-phényl-éthyl)-3-(4-pyrrolidin-1-ylméthyl-benzoylamino)-1H-pyrazolo[4,3-d]thiazole-5-carboxamide | |
| 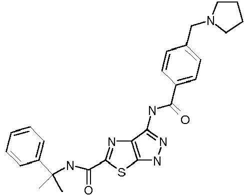 | |

Les composés selon l'invention peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Les composés de l'invention peuvent aussi exister sous forme de sels d'addition. Les sels font également partie de l'invention. Les composés de l'invention comportant un reste basique peuvent être éventuellement transformés en sels d'addition avec un acide minéral ou organique par action d'un tel acide au sein d'un solvant organique tel un alcool, une cétone, un éther ou un solvant chloré. Ces sels sont avantageusement préparés avec des acides pharmaceutiquement acceptables, mais les sels d'autres acides utiles, par exemple, pour la purification ou l'isolement des composés de l'invention font également partie de l'invention. Des sels pharmaceutiquement acceptables incluent les chlorures, nitrates, sulfates, hydrogénosulfates, pyrosulfates, bisulfates, sulfites, bisulfites, phosphates, monohydrogénophosphates, dihydrogénophosphates, métaphosphates, pyrophosphates, acétates, propionates, acrylates, 4-hydroxybutyrates, caprylates, caproates, décanoates, oxalates, malonates, succinates, glutarates, adipates, pimélates, maléates, fumarates, citrates, tartrates, lactates, phénylacétates, mandélates, sébacates, subérates, benzoates, phtalates, méthanesulfonates, propanesulfonates, xylènesulfonates, salicylates, cinnamates, glutamates, aspartates, glucuronates, galacturonates.

Les composés de l'invention comportant un reste acide peuvent être éventuellement transformés en sels métalliques ou en sels d'addition avec des bases azotées selon les méthodes connues en soi. Ces sels peuvent être obtenus par action d'une base métallique (alcaline ou alcalinoterreuse par exemple), de l'ammoniac, d'une amine ou d'un sel d'amine sur un composé de formule (I), dans un solvant. Le sel formé est séparé par les méthodes habituelles. Des bases pharmaceutiquement acceptables incluent des hydroxydes de cations de métaux alcalins ou alcalino-terreux tels que Li, Na, K, Mg, Ca, des composés aminés basiques tels qu'ammoniac, arginine, lysine histidine, pipéridine, morpholine, pipérazine, triéthylamine.

Certains composés selon l'invention sont eux-mêmes prodrogues de composés parents actifs, afin d'augmenter leur biodisponibilité orale selon des méthodes connues de l'homme de l'art.

**Selon un deuxième aspect,** l'invention concerne une composition pharmaceutique comprenant en tant que principe actif un composé selon l'invention, en combinaison avec un excipient pharmaceutiquement acceptable (selon le mode d'administration choisi). La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants. Les formes liquides seront de préférence injectables et, de ce fait, auront une formulation acceptable pour une telle utilisation.

Des voies d'administration par injection acceptables incluent les voies intraveineuse, intra-péritonéale, intramusculaire, et sous cutanée, la voie intraveineuse étant préférée. La dose administrée des composés de l'invention sera adaptée par le praticien en fonction de la voie d'administration du patient et de l'état de ce dernier.

Les composés de la présente invention peuvent être administrés seuls ou en mélange avec au moins un autre anticancéreux. Celui-ci peut être par exemple choisi parmi :
- un agent alkylant et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine ;
- les dérivés du platine comme notamment le cisplatine, le carboplatine ou l'oxaliplatine ;
- les agents antibiotiques comme notamment la bléomycine, la mitomycine, la dactinomycine ;
- les agents antimicrotubules comme notamment la vinblastine, la vincristine, la vindésine, la vinorelbine, les taxoides (paclitaxel et docétaxel) ;
- les anthracyclines comme notamment la doxorubicine, la daunorubicine, l'idarubicine, l'épirubicine, la mitoxantrone, la losoxantrone ;
- les agents inhibiteurs de topoisomérases des groupes I est II telles que l'étoposide, le teniposide, l'amsacrine, l'irinotecan, le topotecan et le tomudex ;
- les fluoropyrimidines telles que le 5-fluorouracile, l'UFT, la floxuridine ;
- les analogues de cytidine telles que la 5-azacytidine, la cytarabine, la gemcitabine, la 6-mercaptomurine, la 6-thioguanine ;
- les analogues d'adénosine telles que la pentostatine, la cytarabine ou le phosphate de fludarabine ;
- le méthotrexate et l'acide folinique ;
- les enzymes et composés divers tels que la L-asparaginase, l'hydroxyurée, l'acide trans-rétinoique, la suramine, la dexrazoxane, l'amifostine, l'herceptin ainsi que les hormones oestrogéniques, androgéniques ;
- les agents antivasculaires tels que les dérivés de la combretastatine ou de la colchicine et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

**Selon un troisième aspect,** l'invention a pour objet l'utilisation d'un composé selon l'invention, pour la fabrication d'un médicament. De préférence, il peut être utile pour traiter le cancer.

**Selon un autre aspect,** l'invention a aussi pour objet le procédé de préparation des composés selon l'invention. Les composés de formule (I) peuvent être préparés à partir des composés de formule (la), selon le Schéma 1 :

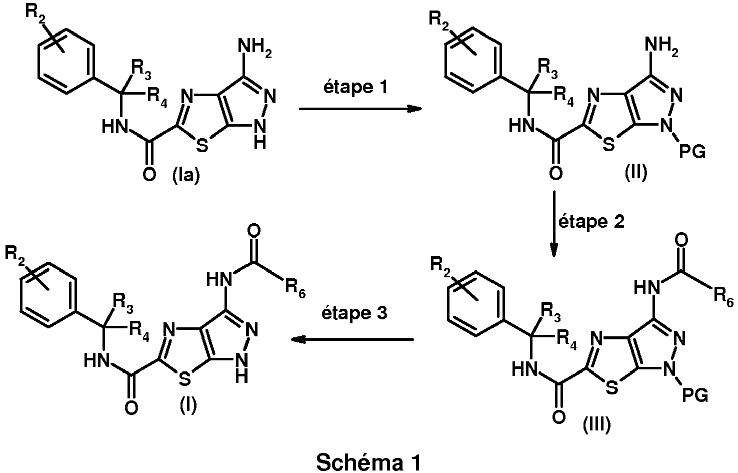

### étape 1

L'étape 1 correspond à une étape de protection du NH du cycle thiazole à l'aide du groupement protecteur PG. La fonction de PG est d'éviter les réactions secondaires non désirées lors d'une ou de plusieurs étape(s) réactionnelles. On trouvera des exemples de groupements protecteurs dans l'ouvrage : T.W. Greene et coll. Protective Groups in Organic Synthesis, third edition, 1999, Wiley-Interscience ou encore dans: J.F.W. McOmie in Protective Groups in Organic Chemistry, Plenum Press, 1973. Comme exemple de groupement protecteur préféré, on peut citer le *tert-*butyloxycarbonyle (BOC) ou le 1-éthoxy-éthyle

Lorsque PG désigne le *tert-*butyloxycarbonyle, l'étape 1 peut s'effectuer avec le di-*tert-*butyldicarbonate en présence d'une base (telle que la triéthylamine ou la pyridine) et éventuellement en présence de N,N-diméthylamino-pyridine, dans un solvant inerte (dichlorométhane par exemple) ou dans la base organique elle-même à une température comprise entre -10°C et la température d'ébullition du milieu réactionnel. Lorsque PG désigne le 1-éthoxy-éthyle, l'étape 1 peut s'effectuer avec l'éthylvinyl éther, en présence d'une quantité catalytique d'un acide tel que l'acide chlorhydrique, au sein d'un solvant inerte tel que le toluène à une température comprise entre 20°C et la température d'ébullition du milieu réactionnel.

### étape 2

La réaction d'acylation de l'étape 2 est conduite avec un agent acylant qui permet d'introduire R₆. Ce dernier peut être par exemple :
- un chlorure d'acide R₆C(O)Cl. La réaction est alors conduite de préférence en présence d'une base, telle que par exemple la triéthylamine, la pyridine, la diisopropyléthylamine, le carbonate de potassium ou de sodium. La réaction peut être conduite dans un solvant inerte (diméthylformamide, tetrahydrofuranne par exemple) ou dans la base organique elle-même à une température comprise entre 0°C et la température débullition du milieu réactionnel (G.Daidone et coll, Heterocycles, 1996, 43(11), 2385) ;
- un anhydride (R₆CO)₂O. La réaction est conduite dans un solvant inerte (diméthylformamide, tétrahydrofuranne, dichlorométhane par exemple) ou dans l'anhydride lui-même à une température comprise entre 0°C et la température d'ébullition du milieu réactionnel (F.Albericio, Synth. Commun., 2001, 31(2), 225, G. Procter, Tetrahedron, 1995, 51(47), 12837) ;
- un acide R₆C(O)H. La réaction est conduite de préférence en présence d'un agent d'activation tel que l'hexafluorophosphate de O-(7-aza-benzotriazol-1-yl)-N,N,N',N'-tétraméthyluronium (HATU) en présence d'une base (pyridine, diisopropyléthylamine ou triéthylamine par exemple) dans un solvant inerte (diméthylformamide par exemple) à une température comprise entre 0°C et la température d'ébullition du milieu réactionnel, ou selon les méthodes bien connues de couplage de la chimie peptidique (M.Bodanszky et coll., Principles of Peptide Synthesis, Springer-Verleg, New York, NY, 1984, 9-58) ou de la formation d'un amide.

### étape 3

Le composé (la) est finalement obtenu après une étape de déprotection (étape 3). Lorsque PG désigne le *tert*-butyloxycarbonyle, l'étape 3 peut s'effectuer en présence d'iodotriméthylsilane, ou en milieu acide (acide trifluoroacétique, ou acide chlorhydrique dans un solvant tel que l'éthanol, le dichlorométhane ou le dioxanne par exemple) à une température comprise entre 0°C et la température d'ébullition du milieu réactionnel, ou bien en milieu basique (carbonate de potassium au sein d'un solvant tel qu'un alcool (méthanol de préférence) à une température comprise entre 0°C et la température d'ébullition du milieu réactionnel et éventuellement sous irradiation par des micro-ondes), ou en milieu neutre dans un solvant tel qu'un alcool (méthanol de préférence) sous irradiation par des micro-ondes. Lorsque PG désigne le groupement 1-éthoxy-éthyle), l'étape 3 peut être conduite en présence d'un acide minéral tel que l'acide chlorhydrique, dans un solvant tel que le tétrahydrofuranne ou l'eau, à une température comprise entre 20°C et latempérature d'ébullition du milieu réactionnel, ou encore selon les méthodes bien connues de déprotection de la fonction amine (T.W. Greene et coll.).

Après l'étape (3), les composés de formule (I) sont isolés et peuvent être purifiés par les méthodes connues habituelles, par exemple par cristallisation, chromatographie ou extraction.

L'invention a donc également pour objet le procédé de préparation d'un composé de formule (Ib) suivante à partir d'un composé de formule (II) : 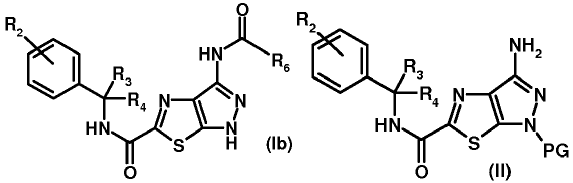 dans lesquelles R₂, R₃, R₄ et R₆ ont les mêmes significations que décrites précédemment et PG désigne un groupement protecteur de la fonction NH du thiazole, et comprenant dans l'ordre les étapes suivantes :
- l'acylation du composé de formule (II) avec un agent acylant permettant d'introduire R₆ (étape 2) ;
- la déprotection du composé issu de l'étape précédente (étape 3).

L'étape 2 est précédée d'une étape de protection de la fonction NH du composé de formule (la) par le groupement protecteur PG (étape 1).

### Préparation des composés de formule (Ia)

Les composés de formule générale (la) peuvent être préparés à partir du 2-triméthylsilyl-thiazole, selon le Schéma 2 :

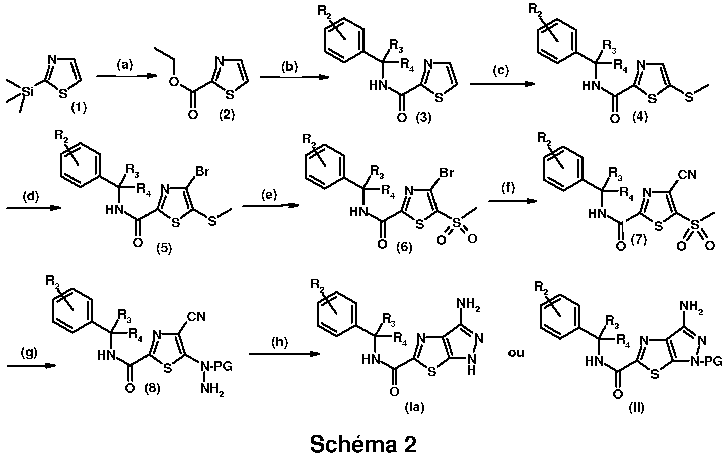

La réaction (a) peut s'effectuer en présence de chloroformiate d'éthyle, dans un solvant inerte tel que le toluène à une température comprise entre 0°C et la température d'ébullition du milieu réactionnel, par adaptation de la méthode décrite dans J. Org. Chem. 1988, 53, 1748.

La réaction d'amidification (b) peut s'effectuer en présence de l'amine de formule (R₂)Ph-C(R₃)(R₄)-NH₂ et de triméthylaluminium dans un solvant tel que le toluène ou le diméthylformamide à une température comprise entre 0°C et la température d'ébullition du milieu réactionnel.

La réaction (c) peut s'effectuer en présence d'une base telle qu'un alkyllithien (le *n-*butyllithium de préférence) et de diméthyldisulfure, dans un solvant inerte tel qu'un éther (tetrahydrofuranne de préférence) à une température comprise entre -78°C et 0°C.

La réaction de bromation (d) peut s'effectuer en présence d'un agent de bromation tel que le N-bromosuccinimide, dans un solvant inerte tel que le chloroforme à une température comprise entre 20°C et la température débullition du milieu réactionnel.

La réaction d'oxydation (e) peut s'effectuer en présence d'OXONE^{®} (peroxymonosulfate de potassium) ou d'acide 3-chloro-peroxybenzoïque, dans un solvant inerte tel que respectivement le tétrahydrofuranne ou le diméthylformamide dans le premier cas ou le dichlorométhane dans le deuxième cas, à une température comprise entre -20°C et la température ambiante.

La réaction (f) peut s'effectuer en présence d'un dérivé de cyanure tel que le cyanure de zinc, d'un dérivé de palladium (0) tel que le tris(dibenzylidène-acétone)dipalladium (0) et d'un ligand tel que le 1,1'-bis-(diphénylphosphino)ferrocène, dans un solvant inerte tel que le diméthylformamide à une température comprise entre 20°C et la température d'ébullition du milieu réactionnel.

Lorsque PG représente un groupe *tert*-butyloxycarbonyle, la réaction (g) peut être conduite en présence de N-(*tert-*butyloxycarbonyl)-hydrazine et d'une base telle que le carbonate de potassium, dans un solvant inerte tel que le diméthylformamide à une température comprise entre 20°C et la température d'ébullition du milieu réactionnel.

La réaction de cyclisation (h) peut s'effectuer en présence d'un acide minéral tel que l'acide chlorhydrique (acide chlorhydrique sec dans le dioxanne de préférence) ou l'acide sulfurique, dans un solvant inerte tel qu'un alcool (éthanol de préférence) à une température comprise entre 20°C et la température d'ébullition du milieu réactionnel. La réaction (h) conduit *in fine* au composé (la) ou bien directement au composé (II) utilisé dans le Schéma 1 (auquel cas, l'étape 1 du Schéma 1 n'est plus nécessaire). On obtient l'un ou l'autre des composés selon les conditions opératoires. Par exemple, dans le cas où PG représente un groupe *tert-*butyloxycarbonyle (groupement protecteur préféré pour le Schéma 2), on a constaté que l'on obtient (la) ou (II) selon la durée de la réaction de cyclisation (h).

Les composés intermédiaires (3), (4), (5), (6), (7) et (8) font également partie de l'invention.

### Variante du procédé du Schéma 1

Pour certains groupes de composés détaillés ci-dessous, on peut utiliser une variante du procédé décrit sur le Schéma 1 s'appuyant sur l'utilisation des composés intermédiaires (IIIa) ou (IIIb) et d'une étape (a) avant l'étape (3) : 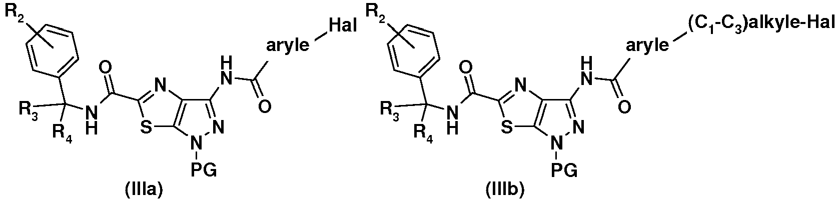 Hal représente un atome d'halogène relié à un groupe aryle ou à un groupe (C₁-C₃)alkyle ayant une fonction de groupe partant et pouvant être un atome de chlore, de brome ou de fluor. Les composés (IIIa) ou (IIIb) peuvent être préparés selon le procédé du Schéma 1.

Cas où R₆ représente un thiényle-2 substitué en position 4 ou 5 par un groupement 4-alkyle-pipérazin-1-yle (en particulier 4-méthyl-pipérazin-1-yle) (Schéma 3) 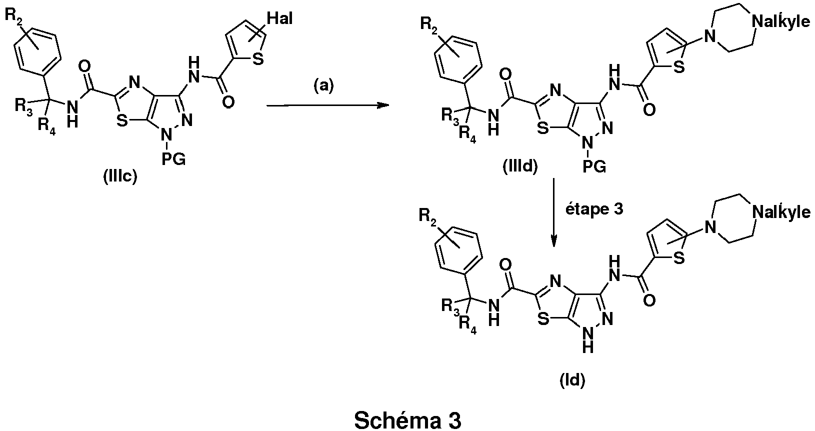 ou bien R₆ représente un pyridyle-2 ou -3 substitué en position 6 ou pyridyle-3 substitué en position 6 par un groupement 4-alkyle-pipérazin-1-yle (en particulier 4-méthyl-pipérazin-1-yle) (Schéma 4)

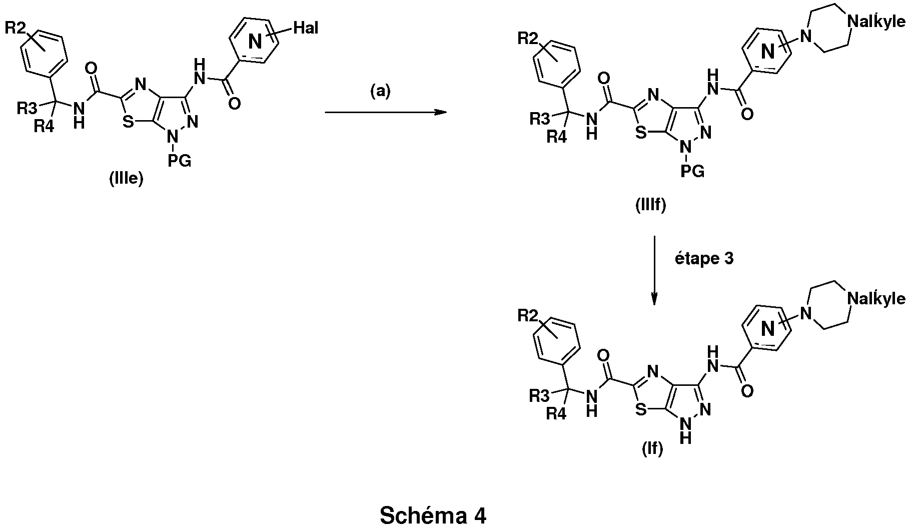

La réaction (a) peut s'effectuer:
- lorsque Hal représente un atome de chlore ou de brome à l'aide de 4-méthyl-pipérazine, en présence d'iodure de cuivre (I), d'une amine telle qu'un acide aminé (la *L-*proline par exemple) ou le *trans*-1,2-diaminocyclohexane ou alternativement d'un diol, et d'une base telle que le carbonate de potassium, le phosphate tripotassique ou le carbonate de césium. On utilise un solvant inerte tel que le dioxanne, le diméthylsulfoxyde ou l'isopropanol à une température comprise entre 20°C et la température débullition du milieu réactionnel, éventuellement sous irradiation par des micro-ondes selon les méthodes générales décrites par D.Ma et al., Synthesis 2005, 496; Org. Letters 2003, 5, 2453 et par S. L.Buchwald et al., Org. Letters 2002, 4, 581 ;
- lorsque Hal représente un atome de chlore ou de brome à l'aide de 4-méthyl-pipérazine, en présence d'un dérivé de palladium(O) (tris(dibenzylidène-acétone)dipalladium (0) par exemple), d'un ligand tel qu'un dérivé de phosphine (2-dicyclohexylphosphino-2'-(N,N-diméthylamino)biphényle ou 2-(di-*t*-butylphosphino)biphényle par exemple), et d'une base, telle que le *tert-*butylate de sodium, le phosphate tripotassique ou le carbonate de césium. On utilise un solvant inerte tel que le toluène ou le diméthoxyéthane, à une température comprise entre 20°C et la température débullition du milieu réactionnel, éventuellement sous irradiation par des micro-ondes, selon les méthodes générales décrites par S.L. Buchwald et al., J.Org.Chem. 2000, 65, 1158; J.Org.Chem. 2000, 65, 1144; Org.Letters 2003, 5, 2413 et par J.G.Verkade et al., J.Org.Chem. 2003, 68, 8416 ;
- lorsque Hal représente un atome de chlore ou de fluor à l'aide de 4-méthyl-pipérazine, en présence d'une base telle que le carbonate de potassium et éventuellement en présence d'un agent complexant tel qu'un éther couronne (18-couronne-6 de préférence). On utilise un solvant inerte tel que le diméthylformamide à une température comprise entre 20°C et la température d'ébullition du milieu réactionnel.

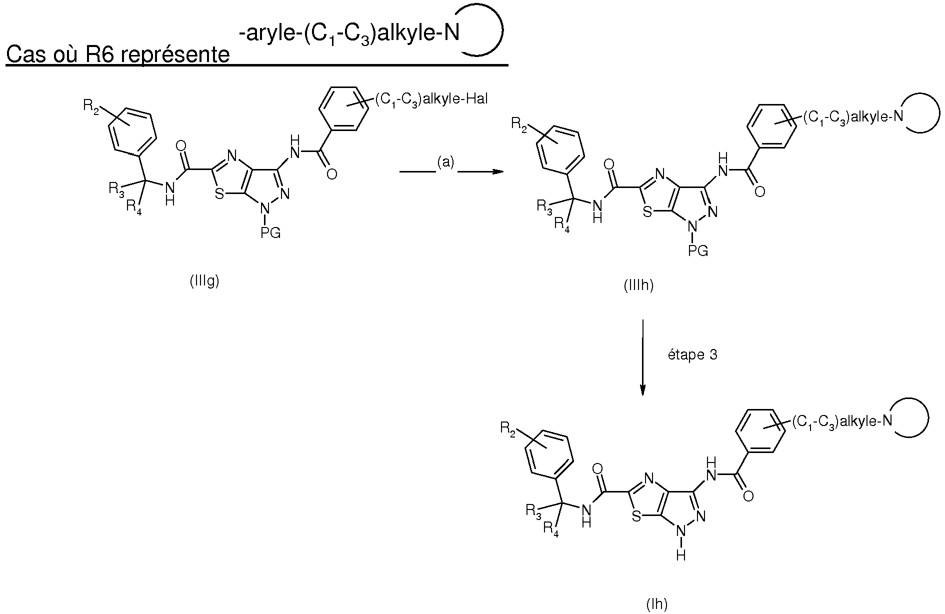

La réaction (a) peut s'effectuer en présence de 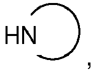 en présence d'un sel tel que l'iodure de tetrabutylammonium au sein d'un solvant inerte tel que le diméthylformamide, à une température comprise entre 20°C et la température d'ébullition du milieu réactionnel.

L'invention a donc aussi pour objet un procédé de préparation des composés (IIIb) à partir des composés (IIIa) comprenant l'étape suivante : 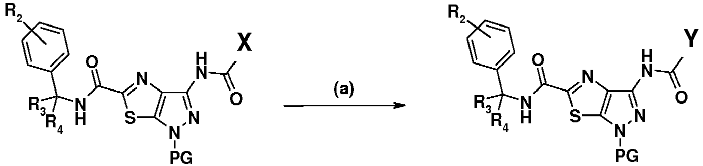 dans laquelle :
**X** représente 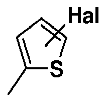 et **Y** représente 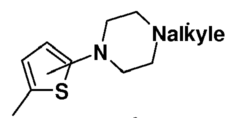 ou bien
**X** représente 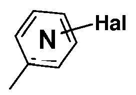 et **Y** représente 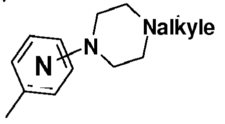 ou bien
**X** représente 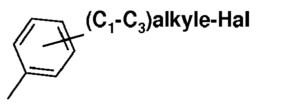 et **Y** représente

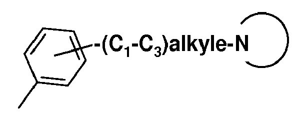

A la suite de l'étape (a), on applique l'étape 3 de déprotection.

Les composés suivants font également partie de l'invention :

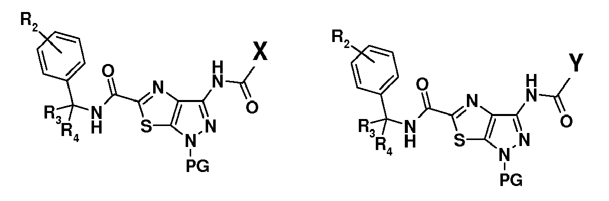

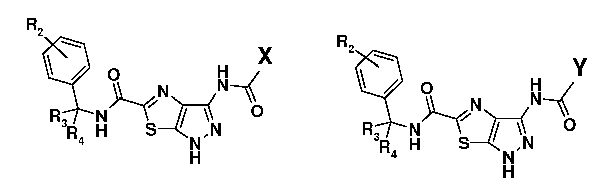

Il est entendu pour l'homme du métier que, pour la mise en oeuvre des procédés selon l'invention décrits précédemment, il peut être nécessaire d'introduire en plus de PG un ou plusieurs autre(s) groupement(s) protecteur(s) permettant de protéger une ou plusieurs autre(s) groupe(s) chimique(s), éliminé(s) par la suite.

### [Exemples]

L'invention est également décrite par les exemples suivants, donnés à titre d'illustration de l'invention.

### Méthodes de caractérisation :

### analyses par LC/MS méthode A

Les analyses LC/MS ont été réalisées sur un appareil HPLC équipé de pompes Shimadzu modèle LC-10AD, d'un passeur d'échantillons Gilson modèle 215, d'un détecteur UV Shimadzu modèle SPD-10A. Les spectres MS ont été réalisés en électrospray (ES⁺/ES⁻) sur un appareil PE Sciex API 100LC. La séparation a été effectuée sur une colonne YMC basic S5 en éluant par un gradient d'acétonitrile contenant 0,1% (v/v) d'acide trifluoroacétique dans l'eau à un débit de 0,1 ml/mn. Les données ont été analysées en utilisant le logiciel Micromass MassLynx. Les principaux ions observés sont décrits.

### analyses par LC/MS méthode B

Les analyses LC/MS ont été réalisées sur un appareil HPLC Agilent modèle HP1100. Les spectres MS ont été réalisés en électrospray (ES⁺/ES⁻) sur un appareil Waters modèle ZQ. La séparation a été effectuée sur une colonne Waters Xbridge C18 (3x50 mm, porosité 2,5 µm) maintenue à une température de 60°C en éluant par un gradient d'acétonitrile dans l'eau contenant 0,1% (v/v) d'acide formique à un débit de 1,1 ml/mn. Le gradient a le profil suivant: 5 à 100% acétonitrile en 5 minutes, 100% acétonitrile constant pendant 0,5 minute, puis retour à 5% acétonitrile en 1 minute. Le temps total d'analyse, incluant la période de rééquilibration de la colonne, est de 7 minutes. L'abondance des produits a été mesurée à l'aide d'un détecteur à barrettes de diodes sur une gamme d'onde de 210 à 400 nm et un détecteur à dispersion de lumière Sedere Sedex 85. L'acquisition des spectres de masse a été réalisée sur une gamme de 100 à 1200 unités de masse atomique. Les données ont été analysées en utilisant le logiciel Micromass MassLynx. Les principaux ions observés sont décrits.

### analyses par LC/MS méthode C

Les analyses LC/MS ont été réalisées sur un appareil HPLC Agilent modèle HP1100. Les spectres MS ont été réalisés en électrospray (ES⁺/ES⁻) sur un appareil Waters modèle ZQ. La séparation a été effectuée sur une colonne Waters Xbridge C18 (3x50 mm, porosité 2,5 µm) maintenue à une température de 70°C en éluant par un gradient d'acétonitrile dans l'eau contenant 0,1% (v/v) d'acide formique à un débit de 0,9 ml/mn. Le gradient a le profil suivant: 5 à 100% acétonitrile en 5 minutes 30 secondes, 100% acétonitrile constant pendant 20 secondes, puis retour à 5% acétonitrile en 40 secondes. Le temps total d'analyse, incluant la période de rééquilibration de la colonne, est de 7 minutes. L'abondance des produits a été mesurée à l'aide d'un détecteur à barrettes de diodes sur une gamme d'onde de 210 à 400 nm et un détecteur à dispersion de lumière Sedere Sedex 85. L'acquisition des spectres de masse a été réalisée sur une gamme de 100 à 1200 unités de masse atomique. Les données ont été analysées en utilisant le logiciel Micromass MassLynx. Les principaux ions observés sont décrits.

### analyses par LC/MS méthode D

Les analyses LC/MS ont été réalisées sur un appareil HPLC Waters modèle Acquity UPLC. Les spectres MS ont été réalisés en électrospray (ES⁺/ES⁻) sur un appareil Waters modèle ZQ. La séparation a été effectuée sur une colonne Waters UPLC BeH C18 (2,1x50 mm, porosité 1,7 µm) maintenue à une température de 55°C en éluant par un gradient d'acétonitrile dans l'eau contenant 0,1% (v/v) d'acide formique à un débit de 1,2 ml/mn. Le gradient a le profil suivant: 5 à 100% acétonitrile en 3 minutes, puis retour à 5% acétonitrile en 1 minute. Le temps total d'analyse, incluant la période de rééquilibration de la colonne, est de 4,5 minutes. L'abondance des produits a été mesurée à l'aide d'un détecteur à barrettes de diodes sur une gamme d'onde de 210 à 400 nm. L'acquisition des spectres de masse a été réalisée sur une gamme de 100 à 1200 unités de masse atomique. Les données ont été analysées en utilisant le logiciel Micromass MassLynx. Les principaux ions observés sont décrits.

### analyses par RMN ¹H

Les analyses ont été réalisées à 400 MHz sur spectromètre BRUKER AVANCE DRX-400 avec les déplacements chimiques (δ en ppm) mesurés dans le diméthylsulfoxide - d6 (DMSO-d6) référencé à 2,50 ppm à la température de 303 K.

### Exemple 1: N-(1-méthyl-1-phényl-éthyl)-(3-amino-1H-pyrazolo[4,3-d]thiazole)-5-carboxamide (R₁=NH₂, R₅=H, R₃=R₄=Me, R₂=H)

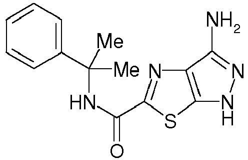

### 1.1 2-thiazolecarboxylate d'éthyle (2):

On charge un ballon de 2 litres sous argon avec 10 g (63,7 mmol) de 2-triméthylsilyl-thiazole (1) et 435 ml de toluène. On agite et refroidit le mélange réactionnel à l'aide d'un bain de glace, puis lorsque la température atteint environ 8°C on ajoute une solution de 12,2 ml (0,128 mol) de chloroformiate d'éthyle dans 500 ml de toluène. Quand la totalité est coulée, on laisse s'écouler environ 10 min avant de laisser la température remonter à 25°C. Après 20 h d'agitation, on coule 200 ml d'une solution aqueuse de carbonate de sodium et on agite pendant 30 min. Après décantation, la phase organique est lavée avec 200 ml d'eau saturée en chlorure de sodium. Après séchage sur sulfate de magnésium, la phase organique est concentrée à sec sous pression réduite. Le résidu est purifié par chromatographie flash (m_{silice}=200 g ; éluant : cyclohexane/acétate d'éthyle : 60/40). On obtient ainsi 5,12 g de 2-thiazolecarboxylate d'éthyle **(2)** sous forme d'un liquide verdâtre qui cristallise. R_{f} (cyclohexane/acétate d'éthyle : 50/50) = 0,52.

### 1.2 N-(1-méthyl-1-phényl-éthyl)-thiazole-2-carboxamide:

Dans un tricol de 1 litre, on introduit 7.13 ml (50.0 mmol) de cumylamine et 156 ml de toluène sous agitation et sous atmosphère inerte. On coule ensuite goutte à goutte 24,8 ml (49,6 mmol) de triméthylaluminium, une fumée blanche se dégage. On attend que le milieu s'éclaircisse puis on coule une solution de 3,9 g (24,8 mmol) de 2-thiazolecarboxylate d'éthyle (2) dans 195 ml de toluène. On laisse agiter pendant ¼ h, puis on chauffe à reflux. Après 15 h de chauffage, on jette le milieu réactionnel sur 300 ml d'une solution d'acide citrique 1 M diluée par 200 ml d'eau et le tout est filtré sur Celite^{®}. On lave la Celite^{®} par 100 ml d'acétate d'éthyle et on extrait la phase aqueuse par 2 fois 100 ml d'acétate d'éthyle. Les extraits organiques sont réunis, séchés sur sulfate de magnésium et après évaporation sous pression réduite on purifie le résidu par chromatographie flash (m_{silice}=200 g ; éluant : dichlorométhane). On obtient ainsi 4,1 g de N-(1-méthyl-1-phényl-éthyl)-thiazole-2-carboxamide sous forme d'un solide rouge brique. R_{f} (dichlorométhane)=0,4.

### 1.3 N-(1-méthyl-1-phényl-éthyl)-(5-méthylsulfanyl-thiazole)-2-carboxamide:

Dans un tricol de 250 ml, on introduit sous argon une solution de 6,1 g (24,8 mmol) de N-(1-méthyl-1-phényl-éthyl)-thiazole-2-carboxamide dans 60 ml de tetrahydrofuranne anhydre préalablement séchée sur tamis moléculaire. Après refroidissement à -78°C sous agitation, on coule doucement à l'aide d'une seringue 31 ml (49,6 mmol) de n-butyllithium. Un précipité marron foncé se forme aussitôt. On laisse agiter pendant 2 heures à -78°C, puis l'on coule 4,4 ml (49,6 mmol) de diméthyle disulfure et au bout d'une heure d'agitation à -78°C, on laisse la température remonter à 25°C et on agite pendant uneheure à cette température. On jette ensuite le milieu réactionnel dans 200 ml d'eau. La phase aqueuse est extraite par 3 fois 100 ml d'acétate d'éthyle. Les extraits organiques sont réunis, séchés sur sulfate de magnésium, puis concentrés à sec sous pression réduite. Le résidu est purifié par chromatographie flash (m_{silice}=200 g ; éluant : cyclohexane/acétate d'éthyle : 75/25). On obtient ainsi 5,2 g de N-(1-méthyl-1-phényl-éthyl)-(5-méthylsulfanyl-thiazole)-2-carboxamide sous forme d'un solide jaune qui cristallise. Pf_{K}=68°C.

### 1.4 N-(1-méthyl-1-phényl-éthyl)-(4-bromo-5-méthylsulfanyl-thiazole)-2-carboxamide:

Dans un tricol de 250 ml, on dissout 5,2 g (17,8 mmol) du N-(1-méthyl-1-phényl-éthyl)-(5-méthylsulfanyl-thiazole)-2-carboxamide dans 50 ml de chloroforme sous argon. On ajoute 3,49 g (19,6 mmol, 1,1 éq) de N-bromosuccinimide et on agite le mélange réactionnel pendant 7 h. On lave ensuite le milieu réactionnel par 3 fois 50 ml d'eau, puis on sèche la phase organique sur sulfate de magnésium et on la concentre à sec sous pression réduite. Le résidu est purifié par chromatographie flash (m_{silice}=200 g ; éluant : cyclohexane/acétate d'éthyle : 80/20). On obtient ainsi 6,5 g N-(1-méthyl-1-phényl-éthyl)-(4-bromo-5-méthylsulfanyl-thiazole)-2-carboxamide sous forme d'une huile jaunâtre. Rf (acétate d'éthyle/cyclohexane : 50/50) = 0,88.

### 1.5 N-(1-méthyl-1-phényl-éthyl)-(4-bromo-5-méthanesulfonyl-thiazole)-2-carboxamide:

Dans un tricol de 500 ml, on dissout 6,5 g (0,018 mol) de N-(1-méthyl-1-phényl-éthyl)-(4-bromo-5-méthylsulfanyl-thiazole)-2-carboxamide dans 250 ml de diméthylformamide sous argon. On refroidit par un bain d'eau et on ajoute ensuite progressivement à la spatule 43 g (0,07 mol, 4 éq) d'Oxone^{®} (peroxymonosulfate de potassium). A la fin de l'addition on enlève le bain et l'on agite le mélange réactionnel sous argon pendant 15 h à 25°C. On rajoute ensuite 11 g (0,018 mol, 1 éq) d'Oxone^{®} et on continue l'agitation pendant 15 h à 25°C. Onrajoute ensuite 11 g (0,018 mol, 1 éq) d'Oxone^{®} et on continue l'agitation pendant 15 h à 25°C. Onjette ensuite le milieu réactionnel dans 300 ml d'eau, puis on extrait par 4 fois 150 ml d'acétate d'éthyle. Après séchage sur sulfate de magnésium, les extraits organiques réunis sont concentrés à sec sous pression réduite. Le résidu est repris par 400 ml d'acétate d'éthyle et lavé par 3 fois 100 ml d'eau. Après séchage sur sulfate de magnésium, la phase organique est concentrée à sec sous pression réduite. On obtient ainsi 6,7 g de N-(1-méthyl-1-phényl-éthyl)-(4-bromo-5-méthanesulfonyl-thiazole)-2-carboxamide sous forme d'un solide crème. Pf_{K}=146°C.

### 1.6 N-(1-méthyl-1-phényl-éthyl)-(4-cyano-5-méthanesulfonyl-thiazole)-2-carboxamide:

Dans un tricol de 250 ml, on dissout 4,5 g (11,2 mmol) du N-(1-méthyl-1-phényl-éthyl)-(4-bromo-5-méthanesulfonyl-thiazole)-2-carboxamide dans 60 ml de diméthylformamide sous argon et sous agitation. On charge ensuite 1,3 g (11,1 mmol) de cyanure de zinc, 1,24 g (2,24 mmol) de 1,1'-bis-(diphénylphosphino)ferrocène (dppf), et 1,13 g (1,23 mmol) de tris(dibenzylidène-acétone)dipalladium (0) (Pd₂(dba)₃). On fait dégazer le mélange sous argon par un tube téflon plongeant dans la solution pendant 2 minutes puis on remonte le tube au dessus de la solution en continuant le balayage d'argon et on chauffe le mélange réactionnel à 120° C pendant 15 h. On jette ensuitele milieu réactionnel dans 200 ml d'eau puis l'on extrait par 200 ml d'acétate d'éthyle. On filtre sur Celite^{®} et on extrait la phase aqueuse par 2 fois 100 ml d'acétate d'éthyle. Les extraits organiques sont réunis, séchés sur sulfate de magnésium puis concentrés à sec sous pression réduite. Le résidu est repris par 300 ml de dichlorométhane et lavé par 5 fois 30 ml d'eau. Après séchage sur sulfate de magnésium, la phase organique est concentrée à sec sous pression réduite. Le résidu est purifié par chromatographie flash (m_{silice}=100 g ; éluant : 98/2 dichlorométhane/méthanol). On obtient ainsi 3,31 g de N-(1-méthyl-1-phényl-éthyl)-(4-cyano-5-méthanesulfonyl-thiazole)-2-carboxamide sous forme d'un solide beige. Pf_{K}=209°C.

### 1.7 N-(tert-butyloxycarbonyl)-N-[4-cyano-2-(1-méthyl-1-phényl-éthylcarbamoyl)-thiazol-5-yl]-hydrazine:

Dans un tricol de 50 ml, on dissout 1,03 g (2,95 mmol) du N-(1-méthyl-1-phényl-éthyl)-(4-cyano-5-méthanesulfonyl-thiazole)-2-carboxamide dans 40 ml de diméthylformamide sous argon. On ajoute ensuite progressivement 0.407 g de carbonate de potassium (2,95 mmol) et 0,42 g (3,18 mmol) de N-*(tert-*butyloxycarbonyl)-hydrazine. Après agitation pendant 15 h à 40°C, on concentre le milieu réactionnel à sec sous pression réduite (bain de chauffage 65°C max). Le résidu est repris par 40 ml de dichlorométhane et 40 ml d'eau et l'émulsion obtenue est cassée par l'addition de sel. La phase organique est décantée, séchée sur sulfate de magnésium puis concentrée à sec sous pression réduite. Le résidu est repris par 50 ml de dichlorométhane et 30 ml d'eau. L'émulsion obtenue est cassée par l'addition de sel. La phase organique est décantée, séchée sur sulfate de magnésium puis concentrée à sec sous pression réduite. Le résidu est purifié par chromatographie flash (m_{silice}=50 g ; éluant : 98/2 dichlorométhane/méthanol). On obtient ainsi 0,308 g de N-(*tert*-butyloxycarbonyl)-N-[4-cyano-2-(1-méthyl-1-phényl-éthylcarbamoyl)-thiazol-5-yl]-hydrazine sous forme d'une meringue jaune. Pf_{K}=70°C.

### 1.8 N-(1-méthyl-1-phényl-éthyl)-(3-amino-1H-pyrazolo[4,3-d]thiazole)-5-carboxamide:

Dans un ballon de 50 ml, on dissout 0,083 g (0,21 mmol) du N-(*tert-*butyloxycarbonyl)-N-[4-cyano-2-(1-méthyl-1-phényl-éthylcarbamoyl)-thiazol-5-yl]-hydrazine dans 5 ml d'éthanol. On ajoute ensuite progressivement 1 ml (4,0 mmol) d'une solution d'acide chlorhydrique 4 M dans le dioxanne et l'on agite pendant 15 h à 25°C. Le milieu réactionnel est ensuite concentré à sec sous pression réduite (40°C) et le résidu est repris par 20 ml de dichlorométhane et 20 ml d'eau. La phase aqueuse est alcalinisée par une solution d'hydroxyde de sodium 0,1 N. Après décantation, la phase organique est séchée sur sulfate de magnésium et concentrée à sec sous pression réduite. Le résidu est purifié par chromatographie flash (m_{silice}=30 g ; éluant : 90/10 dichlorométhane/méthanol). On obtient ainsi 28 mg de N-(1-méthyl-1-phényl-éthyl)-(3-amino-1H-pyrazolo[4,3-*d*]thiazole)-5-carboxamide sous forme d'une meringue jaune. Pf_{K}=110°C. R(dichlorométhane/méthanol 90/10)=0,48. RMN ¹H (400 MHz, DMSO d6) : δ ppm 1,70 (s, 6H) ; 6,17 (m étalé, 2H) ; 7,21 (t, J=7,5 Hz, 1 H); 7,32 (t, J=7,5 Hz, 2H) ; 7,42 (d large, J=7,5 Hz, 2H) ; 8,10 (m étalé, 1 H) ; 12,2 (m étalé, 1 H).

### Exemple 2: N-(1-méthyl-1-phényl-éthyl)-3-[4-(4-méthyl-pipérazin-1-yl)-benzoylamino]-1H-pyrazolo[4,3-d]thiazole-5-carboxamide

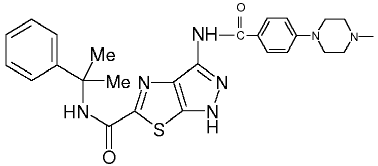

### 2.1 3-amino-5-(1-méthyl-1-phényl-éthylcarbamoyl)-1H-pyrazolo[4,3-d]thiazole-1-carboxylate de tert-butyle

Dans un ballon de 100 ml, on dissout 0,115 g (0,38 mmol) du N-(1-méthyl-1-phényl-éthyl)-(3-amino-1H-pyrazolo[4,3-*d*]thiazole)-5-carboxamide dans 5 ml de pyridine. On ajoute ensuite progressivement 0,092 g (0,42 mmol) de di-*tert*-butyl-dicarbonate et l'on agite le mélange réactionnel pendant 15 h à 25°C. Le milieu réactionnel est ensuite concentré à sec sous pression réduite (40°C) et le résidu est repris par 40 ml d'acétate d'éthyle et lavé par 3 fois 20 ml d'eau. La phase organique est séchée sur sulfate de magnésium puis concentrée à sec sous pression réduite. On obtient ainsi 0,144 g de 3-amino-5-(1-méthyl-1-phényl-éthylcarbamoyl)-1H-pyrazolo[4,3-*d*]thiazole-1-carboxylate de *tert*-butyle sous forme d'une meringue jaune. LC-MS-DAD-ELSD : t_{R} = 4,7 min, m/z = 402.

### 2.2 5-(1-méthyl-1-phényl-éthylcarbamoyl)-3-[4-(4-méthyl-pipérazin-1-yl)-benzoylamino]-1H-pyrazolo[4,3-d]thiazole-1-carboxylate de tert-butyle

Dans un ballon de 250 ml, on introduit 2,0 g (5,0 mmol) de 3-amino-5-(1-méthyl-1-phényl-éthylcarbamoyl)-1H-pyrazolo[4,3-d]thiazole-1-carboxylate de *tert-*butyle qui est dissous dans 40 ml de pyridine sous argon. On ajoute ensuite progressivement 1.56 g (5,0 mmol) de chlorure de 4-(4-méthylpiperazin-1-yl)-benzoyle; dichlorhydrate (préparé selon WO 2005/113494**)** et l'on agite pendant 15 h à 25°C. Le milieu réactionnel est ensuite concentré à sec sous pression réduite (40°C) et le résidu est repris par 50 ml de dichlorométhane et 50 ml d'eau que l'on alcalinise par une solution d'hydroxyde de sodium 0,1 N. L'émulsion obtenue est résorbée par saturation avec du chlorure de sodium. Après décantation, la phase organique est séchée sur sulfate de magnésium et concentré à sec sous pression réduite. Le résidu est purifié par chromatographie flash (m_{silice}=30 g ; éluant : 95/5 dichlorométhane/méthanol). On obtient ainsi 1,32 g de 5-(1-méthyl-1-phényl-éthylcarbamoyl)-3-[4-(4-méthyl-pipérazin-1-yl)-benzoylamino]-1H-pyrazolo[4,3-*d*]thiazole-1-carboxylate de *tert*-butyle sous forme d'une meringue jaune. RMN-¹H (400 MHz, DMSO d6) : δ ppm 1,62 (s, 9H) ; 1,70 (s, 6H) ; 2,22 (s, 3H) ; 2,44 (m, 4H) ; 3,32 (m, 4H) ; 7,02 (d, J=8,5 Hz, 2H) ; 7,20 (t, J=7,5 Hz, 1 H) ; 7,30 (t, J = 7,5 Hz, 2H) ; 7,40 (d, J=7,5 Hz, 2H) ; 7,95 (d, J=8,5 Hz, 2H) ; 8,71 (s, 1 H) ; 11,05 (s, 1 H). LC-MS-DAD-ELSD : 602(-)=(M-H)(-) ; 604(+)=(M+H)(+).

### 2.3 N-(1-méthyl-1-phényl-éthyl)-3-[4-(4-méthyl-pipérazin-1-yl)-benzoylamino]-1H-pyrazolo[4,3-d]thiazole-5-carboxamide:

Dans un ballon de 500 ml, on introduit 2,47 g (4,1 mmol) du 5-(1-méthyl-1-phényl-éthylcarbamoyl)-3-[4-(4-méthyl-pipérazin-1-yl)-benzoylamino]-1H-pyrazolo[4,3-*d*]thiazole-1-carboxylate de *tert-*butyle qui est dissous dans 120 ml d'éthanol. On coule ensuite au goutte à goutte 21 ml (84 mmol) d'une solution d'acide chlorhydrique dans le dioxanne 4 M et l'on agite le mélange réactionnel pendant 15 h à 25°C. On rajoute ensuite 21 ml (84 mmol) de solution d'acide chlorhydrique dans le dioxanne 4 M et l'on agite pendant 15 h à 25°C. Le milieu réactionnel est alors concentré à sec sous pression réduite (40°C) et le résidu est repris par 500 ml de dichlorométhane et 500 ml d'eau que l'on alcalinise par une solution d'hydroxyde de sodium 0,1 N. L'émulsion obtenue est résorbée par saturation avec du chlorure de sodium. Lors de la décantation, on isole par filtration 1,27 g d'un solide blanc cassé qui est purifié par chromatographie flash (m_{silice}=30 g; éluant: 90/10 dichlorométhane/méthanol). On obtient ainsi 0,92 g de N-(1-méthyl-1-phényl-éthyl)-3-[4-(4-méthyl-pipérazin-1-yl)-benzoylamino]-1H-pyrazolo[4,3-*d*]thiazole-5-carboxamide sous forme d'une meringue jaune. Après décantation, la phase organique est séchée sur sulfate de magnésium et concentrée à sec sous pression réduite (40°C). Le résidu jaune est purifié par chromatographie flash (m_{silice}=30 g ; éluant: 90/10 dichlorométhane/méthanol). On obtient ainsi 0,61 g de N-(1-méthyl-1-phényl-éthyl)-3-[4-(4-méthyl-pipérazin-1-yl)-benzoylamino]-1H-pyrazolo[4,3-*d*]thiazole-5-carboxamide sous forme d'une meringue jaune. Les deux lots sont réunis, triturés dans 20 ml d'éther diéthylique, filtrés et séchés sous pression réduite (40°C). On obtient ainsi 1,45 g de N-(1-méthyl-1-phényl-éthyl)-3-[4-(4-méthyl-pipérazin-1-yl)-benzoylamino]-1H-pyrazolo[4,3-*d*]thiazole-5-carboxamide sous forme d'un solide jaune. Pf_{K}=264°C. RMN-¹H (400 MHz, DMSO d6) : δ ppm 1,71 (s, 6H) ; 2,22 (s, 3H) ; 2,45 (m, 4H) ; 3,32 (m, 4H) ; 7,02 (d, J=8,5 Hz, 2H) ; 7,20 (t, J=7,5 Hz, 1 H) ; 7,31 (t, J=7,5 Hz, 2H) ; 7,41 (d, J=7,5 Hz, 2H) ; 7,97 (d, J=8,5 Hz, 2H) ; 8,49 (s large, 1 H) ; 10,8 (m étalé, 1 H) ; 13,5 (m, étalé, 1 H).

### Exemple 3: N-(1-méthyl-1-phényl-éthyl)-3-(4-morpholin-4-yl-benzoylamino)-1H-pyrazolo[4,3-d]thiazole-5-carboxamide

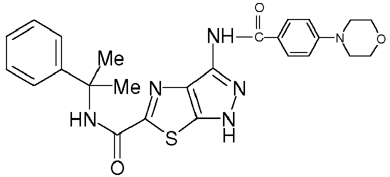

### 3.1 5-(1-méthyl-1-phényl-éthylcarbamoyl)-3-(4-morpholin-4-yl-benzoylamino)-1H-pyrazolo[4,3-d]thiazole-1-carboxylate de tert-butyle:

Dans un ballon de 50 ml, on introduit 0,40 g (0.998 mmol) de 3-amino-5-(1-méthyl-1-phényl-éthylcarbamoyl)-1H-pyrazolo[4,3-*d*]thiazole-1-carboxylate de *tert-*butyle qui est dissous dans 10 ml de pyridine sous argon. On ajoute ensuite progressivement 0,25 g (0,996 mmol) de chlorure de 4-(morpholin-4-yl)-benzoyle; dichlorhydrate (préparé selon WO 95/04729**)** et l'on agite pendant 15 h à 25°C. Le milieu réactionnel est ensuite concentré à sec sous pression réduite (40°C) et le résidu est repris par 50 ml de dichlorométhane et 50 ml d'eau que l'on alcalinise par une solution d'hydroxyde de sodium 0,1 N. L'émulsion obtenue est résorbée par saturation avec du chlorure de sodium. Après décantation, la phase organique est séchée sur sulfate de magnésium et concentré à sec sous pression réduite. Le résidu est purifié par chromatographie flash (m_{silice}=30 g ; éluant : 99/1 dichlorométhane/méthanol). On obtient ainsi 0,19 g de 5-(1-méthyl-1-phényl-éthylcarbamoyl)-3-(4-morpholin-4-yl-benzoylamino)-1H-pyrazolo[4,3-*d*]thiazole-1-carboxylate de *tert*-butyle sous forme d'une meringue jaune. RMN-¹H (400 MHz, DMSO d6) : δ ppm 1,61 (s, 9H) ; 1,70 (s, 6H) ; 3,29 (m masqué, 4H) ; 3,75 (m, 4H) ; 7,03 (d, J=8,5 Hz, 2H) ; 7,20 (t, J=7,5 Hz, 1H) ; 7,30 (t, J=7,5 Hz, 2H) ; 7,40 (d, J=7,5 Hz, 2H) ; 7,97 (d, J=8,5 Hz, 2H) ; 8,69 (s, 1 H) ; 11,1 (m étalé, 1 H). LC-MS-DAD-ELSD : 591 (+)=(M+H)(+) ; 535(+)= 591 (+)-tBu+H.

### 3.2 N-(1-Méthyl-1-phényl-éthyl)-3-(4-morpholin-4-yl-benzoylamino)-1H-pyrazolo[4,3-d]thiazole-5-carboxamide:

Dans un ballon de 50 ml, on introduit 0,19 g (0,32 mmol) du 5-(1-méthyl-1-phényl-éthylcarbamoyl)-3-(4-morpholin-4-yl-benzoylamino)-1H-pyrazolo[4,3-d]thiazole-1-carboxylate de *tert*-butyle qui est dissous dans 7 ml d'éthanol. On coule ensuite 1,6 ml (6,4 mmol) d'une solution d'acide chlorhydrique dans le dioxanne 4 M et l'on agite le mélange réactionnel pendant 15 h à 25°C. On rajoute ensuite 1,6 ml (6,4 mmol) de solution d'acide chlorhydrique dans le dioxanne 4 M et l'on agite pendant 15 h à 25°C. Le milieu réactionnel est alors concentré à sec sous pression réduite (40°C) et le résidu est repris par 40 ml de dichlorométhane et 40 ml d'eau que l'on alcalinise par une solution d'hydroxyde de sodium 0,1 N. L'émulsion obtenue est résorbée par saturation avec du chlorure de sodium. Après décantation, la phase organique est séchée sur sulfate de magnésium et concentrée à sec sous pression réduite (40°C). Le résidu jaune est purifié par chromatographie flash (m_{silice}=30 g ; éluant: 97/3 dichlorométhane/méthanol). On obtient ainsi 0,097 g de N-(1-méthyl-1-phényl-éthyl)-3-(4-morpholin-4-yl-benzoylamino)-1H-pyrazolo[4,3-*d*]thiazole-5-carboxamide sous forme d'une meringue jaune. Pf_{K}=266°C. RMN-¹H (400 MHz, DMSO d6) : δ ppm 1,70 (s, 6H) ; 3,29 (m masqué, 4H) ; 3,75 (m, 4H) ; 7,04 (d, J=8,5 Hz, 2H) ; 7,20 (t, J=7,5 Hz, 1 H) ; 7,31 (t, J=7,5 Hz, 2H) ; 7,41 (d, J=7,5 Hz, 2H) ; 7,99 (d, J=8,5 Hz, 2H) ; 8,50 (s large, 1 H) ; 10,8 (m étalé, 1 H) ; 13,6 (m étalé, 1 H).

### Exemple 4: N-(1-méthyl-1-phényl-éthyl)-3-(4-méthoxy-benzoylamino)-1-H-pyrazolo[4,3-d]thiazole-5-carboxamide

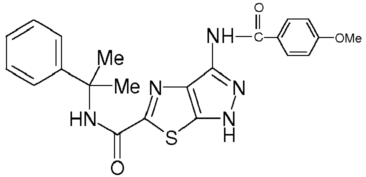

### 4.1 3-(4-méthoxy-benzoylamino)-5-(1-méthyl-1-phényl-éthylcarbamoyl)-1H-pyrazolo[4,3-d]thiazole-1-carboxylate de tert-butyle:

Dans un ballon de 50 ml, on introduit 0,20 g (0,50 mmol) de 3-amino-5-(1-méthyl-1-phényl-éthylcarbamoyl)-1H-pyrazolo[4,3-*d*]thiazole-1-carboxylate de *tert-*butyle qui est dissous dans 5 ml de pyridine sous argon. On ajoute ensuite au goutte à goutte une solution de 0,09 g (0,53 mmol) de chlorure de 4-méthoxy-benzoyle (préparé selon J. Org. Chem. 1976, 41, 2566) dans 2,0 ml de tetrahydrofuranne et l'on agite pendant 15 h à 25°C. Le milieu réactionnel est ensuite concentré à sec sous pression réduite (40°C) et le résidu est repris par30 ml de dichlorométhane et 30 ml d'eau que l'on alcalinise par une solution d'hydroxyde de sodium 0,1 N. L'émulsion obtenue est résorbée par saturation avec du chlorure de sodium. Après décantation, la phase organique est séchée sur sulfate de magnésium et concentré à sec sous pression réduite. Le résidu est purifié par chromatographie flash (m_{silice}=30 g ; éluant : 95/5 dichlorométhane/méthanol). On obtient ainsi 0,05 g de 3-(4-méthoxy-benzoylamino)-5-(1-méthyl-1-phényl-éthylcarbamoyl)-1H-pyrazolo[4,3-*d*]thiazole-1-carboxylate de *tert*-butyle sous forme d'une meringue crème. On obtient par ailleurs 0,16 g d'un lot impur qui est repurifié par chromatographie flash (m_{silice}=30 g ; éluant : 95/5 dichlorométhane/méthanol). On obtient ainsi 0,04 g de 3-(4-méthoxy-benzoylamino)-5-(1-méthyl-1-phényl-éthylcarbamoyl)-1H-pyrazolo[4,3-*d*]thiazole-1-carboxylate de *tert*-butyle sous forme d'une meringue crème. RMN-¹H (400 MHz, DMSO d6) : δ ppm 1,61 (s, 9H) ; 1,71 (s, 6H) ; 3,86 (s, 3H) ; 7,10 (d, J=8,5 Hz, 2H) ; 7,20 (t, J=7,5 Hz, 1 H) ; 7,30 (t, J=7,5 Hz, 2H) ; 7,40 (d, J=7,5 Hz, 2H) ; 8,07 (d, J=8,5 Hz, 2H) ; 8,71 (s, 1 H) ; 11,25 (s large, 1 H). LC-MS-DAD-ELSD : 534(-)=(M-H)(-) ; 536(+)=(M+H)(+).

### 4.2 N-(1-Méthyl-1-phényl-éthyl)-3-(4-méthoxy-benzoylamino)-1-H-pyrazolo[4,3-d]thiazole-5-carboxamide :

Dans un ballon de 100 ml, on introduit 0,16 g (0,31 mmol) du 3-(4-méthoxy-benzoylamino)-5-(1-méthyl-1-phényl-éthylcarbamoyl)-1H-pyrazolo[4,3-*d*]thiazole-1-carboxylate de *tert*-butyle qui est dissous dans 7 ml d'éthanol. On coule ensuite 1,5 ml (6,0 mmol) d'une solution d'acide chlorhydrique dans le dioxanne 4 M et l'on agite le mélange réactionnel pendant 15 h à 25°C. On rajoute ensuite 1,5 ml (6,0 mmol) de solution d'acide chlorhydrique dans le dioxanne 4 M et l'on agite pendant 15 h à 25°C. Le milieu réactionnel est alors concentré à sec sous pression réduite (40°C) et le résidu est repris par 30 ml de dichlorométhane et 30 ml d'eau que l'on alcalinise par une solution d'hydroxyde de sodium 0,1 N. L'émulsion obtenue est résorbée par saturation avec du chlorure de sodium. Après décantation, la phase organique est séchée sur sulfate de magnésium et concentrée à sec sous pression réduite (40°C). Le résidu est purifié par chromatographie flash (m_{silice}=30 g ; éluant: 95/5 dichlorométhane/méthanol). On obtient ainsi 0,103 g de *N*-(1-méthyl-1-phényl-éthyl)-3-(4-méthoxy-benzoylamino)-1-H-pyrazolo[4,3-*d*]thiazole-5-carboxamide sous forme d'une meringue crème. Pf_{K}=226°C. RMN-¹H (400MHz, DMSO d6) : δ ppm 1,70 (s, 6H) ; 3,86 (s, 3H) ; 7,09 (d, J=8,5 Hz, 2H) ; 7,20 (t, J=7,5 Hz, 1 H) ; 7,30 (t, J=7,5 Hz, 2H) ; 7,41 (d, J=7,5 Hz, 2H) ; 8,07 (d, J=8,5 Hz, 2H) ; 8,53 (s large, 1 H) ; de 10,0 à 15,0 (m très étalé, 2H).

### Exemple 5: N-(1-méthyl-1-phényl-éthyl)-3-[(thiophène-3-carbonyl)-amino]-1H-pyrazolo[4,3-d]thiazole-5-carboxamide

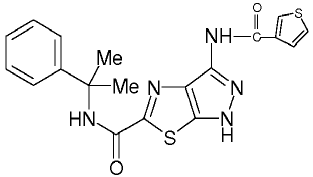

### 5.1 5-(1-Méthyl-1-phényl-éthylcarbamoyl)-3-[(thiophène-3-carbonyl)-amino]-1H-pyrazolo[4,3-d]thiazole-1-carboxylate de tert-butyle :

Dans un ballon de 50 ml, on introduit 0,20 g (0,50 mmol) de 3-amino-5-(1-méthyl-1-phényl-éthylcarbamoyl)-1H-pyrazolo[4,3-*d*]thiazole-1-carboxylate de *tert*-butyle qui est dissous dans 5 ml de pyridine sous argon. On ajoute ensuite au goutte à goutte une solution de 0,07 g (0,50 mmol) de chlorure de thiophène-3-carbonyle (préparé selon FR 2411188) dans 2,0 ml de tetrahydrofuranne et l'on agite pendant 15 h à 25°C. Le milieu réactionnel est ensuite concentré àsec sous pression réduite (40°C) et le résidu est repris par 30 ml de dichlorométhane et 30 ml d'eau que l'on alcalinise par une solution d'hydroxyde de sodium 0,1 N. L'émulsion obtenue est résorbée par saturation avec du chlorure de sodium. Après décantation, la phase organique est séchée sur sulfate de magnésium et concentré à sec sous pression réduite. Le résidu est purifié par chromatographie flash (m_{silice}=30 g ; éluant : 95/5 dichlorométhane/méthanol). On obtient ainsi 0,074 g de 5-(1-méthyl-1-phényl-éthylcarbamoyl)-3-[(thiophène-3-carbonyl)-amino]-1 H-pyrazolo[4,3-*d*]thiazole-1-carboxylate de *tert*-butyle sous forme d'une meringue crème. On obtient par ailleurs 0,14 g d'un lot impur qui est repurifié par chromatographie flash (m_{silice}=30 g ; éluant : 95/5 dichlorométhane/méthanol). On obtient ainsi 0,013 g de 5-(1-méthyl-1-phényl-éthylcarbamoyl)-3-[(thiophène-3-carbonyl)-amino]-1 H-pyrazolo[4,3-*d*]thiazole-1-carboxylate de *tert*-butyle sous forme d'une meringue crème et 0,11 g d'un lot impur qui est repurifié par chromatographie flash (m_{silice}=30 g ; éluant : 95/5 dichlorométhane/méthanol). On obtient ainsi 0,04 g de 5-(1-méthyl-1-phényl-éthylcarbamoyl)-3-[(thiophène-3-carbonyl)-amino]-1 H-pyrazolo[4,3-*d*]thiazole-1-carboxylate de *tert*-butyle sous forme d'une meringue crème. RMN-¹H (400 MHz, DMSO d6) : δ ppm 1,62 (s, 9H) ; 1,71 (s, 6H) ; 7,20 (t, J=7,5 Hz, 1 H) ; 7,30 (t, J=7,5 Hz, 2H) ; 7,41 (d, J=7,5 Hz, 2H) ; 7,70 (d, J=2,0 Hz, 2H) ; 8,50 (t, J=2,0 Hz, 1H) ; 8,70 (s, 1 H) ; 11,25 (s, 1 H). LC-MS-DAD-ELSD : 510(-)=(M-H)(-) ; 512(+)=(M+H)(+).

### 5.2 N-(1-Méthyl-1-phényl-éthyl)-3-[(thiophène-3-carbonyl)-amino]-1H-pyrazolo[4,3-d]thiazole-5-carboxamide :

Dans un ballon de 100 ml, on introduit 0,16 g (0,32 mmol) du 5-(1-méthyl-1-phényl-éthylcarbamoyl)-3-[(thiophène-3-carbonyl)-amino]-1H-pyrazolo[4,3-*d*]thiazole-1-carboxylate de *tert*-butyle qui est dissous dans 7 ml d'éthanol. On coule ensuite 1,6 ml (6,4 mmol) d'une solution d'acide chlorhydrique dans le dioxanne 4 M et l'on agite le mélange réactionnel pendant 15 h à 25°C. On rajoute ensuite 1,6 ml (6,4 mmol) de solution d'acide chlorhydrique dans le dioxanne 4 M et l'on agite pendant 15 h à 25°C. Le milieu réactionnel est alors concentré à sec sous pression réduite (40°C) et le résidu est repris par 30 ml de dichlorométhane et 30 ml d'eau que l'on alcalinise par une solution d'hydroxyde de sodium 0,1 N. L'émulsion obtenue est résorbée par saturation avec du chlorure de sodium. Après décantation, la phase organique est séchée sur sulfate de magnésium et concentrée à sec sous pression réduite (40°C). Le résidu est purifié par chromatographie flash (m_{silice}=30 g ; éluant: 95/5 dichlorométhane/méthanol). On obtient ainsi 0,104 g de N-(1-méthyl-1-phényl-éthyl)-3-[(thiophène-3-carbonyl)-amino]-1H-pyrazolo[4,3-*d*]thiazole-5-carboxamide sous forme d'une meringue crème. Pf_{K}=221°C. RMN-¹H (400 MHz, DMSO d6) : δ ppm 1,70 (s, 6H) ; 7,20 (t, J=7,5 Hz, 1 H) ; 7,31 (t, J=7,5 Hz, 2H) ; 7,41 (d, J=7,5 Hz, 2H) ; 7,69 (m, 2H) ; 8,46 (s large, 1 H) ; 8,53 (s large, 1 H) ; de 10,0 à 15,0 (m très étalé, 2H).

### Activité inhibitrice

De nombreuses protéines impliquées dans la ségrégation des chromosomes et l'assemblage du fuseau ont été identifiées dans la levure et la drosophile. La désorganisation de ces protéines conduit à la non ségrégation des chromosomes et à des fuseaux monopolaires ou désorganisés. Parmi ces protéines, certaines kinases, dont Aurora et Ipl1, provenant respectivement de drosophile et de S. *cerevisiae,* sont nécessaires pour la ségrégation des chromosomes et la séparation du centrosome. Un analogue humain de Ipl1 de levure a été récemment cloné et caractérisé par différents laboratoires. Cette kinase, nommée Aurora 2, STK15 ou BTAK appartient à la famille des kinases à sérine/thréonine. Bischoff et al. ont montré que Aurora 2 est oncogène, et est amplifié dans les cancers colorectaux humains (EMBO J, 1998, 17, 3052-3065). Cela a également été exemplifié dans des cancers impliquant des tumeurs épithéliales telles que le cancer du sein.

Tie2 (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïetine 1 ou Ang1) qui stimule l'autophosphorylation du récepteur et la signalisation cellulaire [S. Davis et al (1996) Cell 87, 1161-1169*]* et l'antagoniste (angiopoïetine 2 ou Ang2) [P.C. Maisonpierre et al. (1997) Science 277, 55-60*].* L'angiopoïetine 1 peut synergiser avec le VEGF dans les derniers stades de la néo-angiogénèse [AsaharaT. Circ. Res.(1998) 233-240]*.* Les expériences de knock-out et les manipulations transgéniques de l'expression de Tie2 ou de Ang1 conduisent à des animaux qui présentent des défauts de vascularisation [D.J. Dumont et al (1994) Genes Dev. 8, 1897-1909 *et* C. Suri (1996) Cell 87, 1171-1180]. La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P.C. Maisonpierre et al (1997) Science 277, 55-60]. Lin et al (1997) J. Clin. Invest. 100, 8: 2072-2078 *et* Lin P. (1998) PNAS 95, 8829-8834*,* ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénogreffes de tumeur du sein et de mélanome.

Les inhibiteurs de Tie2 peuvent être utilisés dans les situations où une néovascularisation se fait de façon inappropriée (c'est-à-dire dans la rétinopathie diabétique, l'inflammation chronique, le psoriasis, le sarcome de Kaposi, la néovascularisation chronique due à la dégénération maculaire, l'arthrite rhumatoïde, l'hémoangiome infantile et les cancers).

### Détermination de l'inhibition des kinases Aurora 1 et Aurora 2

L'effet inhibiteur de composés vis-à-vis des kinases Aurora 1 et Aurora 2 est déterminé par un test enzymatique utilisant une détection de radioactivité. L'activité kinase de Aurora 1 et Aurora 2 est évaluée par la phosphorylation du substrat Numa-histidine en présence d'ATP radiomarqué ([³³P]ATP) en utilisant des plaques 96 puits Flash plate où le nickel-chelate est fixé à la surface de la microplaque. La quantité de phosphate ³³P incorporé au substrat NuMA est proportionnelle à l'activité de l'enzyme Aurora 1 ou Aurora 2.
Aurora 1 : complexe recombinant Aurora-B/INCENP-C3, purifié à environ 50% dont l'extrémité N-terminale de Aurora-B a été marquée à l'histidine.
Aurora 2 : protéine recombinante entière comprenant une queue histidine en N-terminal, a été exprimée dans E.coli et purifiée à plus de 82 %.
NuMA (protéine nucléaire qui s'associe avec l'appareil mitotique) : fragment de 424 acides amines, exprimé dans E.coli dont l'extrémité N-terminale a été marquée à l'histidine et utilisé comme substrat pour les deux enzymes Aurora.

Les microplaques utilisées sont des plaques Flash-Plate, 96 puits, nickel chélate ( Perkin Elmer, modèle SMP107). Les produits à évaluer sont incubés dans un volume réactionnel de 100 µl par puits, en présence de 10 nM de Aurora 1 ou Aurora 2, 500 nM de substrat NuMA dans un tampon composé de 50 mM de Tris/HCl (pH 7,5), 50 mM NaCl, 5 mM MgCl₂ (Aurora-B) ou 10 mM MgCl₂ (Aurora-A) et 1 mM de DTT, à 37°C. Dans chaque puits, 80 µl du tampon d'hcubation enzyme/substrat sont distribués puis 10 µl du produit à évaluer, en concentrations variables. La réaction est initiée par addition de 1µM d'ATP final contenant 0,2 µCi de [³³P]ATP (10 µl). Après 30 minutes d'incubation, la réaction est arrêtée par simple élimination du tampon réactionnel et chaque puits est lavé deux fois avec 300 µl du tampon Tris/HCl. La radioactivité est alors mesurée dans chaque puits à l'aide d'un appareil à scintillation, modèle Packard, Top count.

L'activité enzymatique contrôle d'Aurora est exprimée par le nombre de coups par minute obtenu en 30 minutes après déduction du bruit de fond (mélange réactionnel ne contenant pas l'enzyme). L'évaluation des divers produits testés est exprimée en pourcentage d'inhibition de l'activité Aurora par rapport au contrôle. Dans ce test, les composés inhibent les kinases Aurora 1 et Aurora 2, à des concentrations généralement comprises entre 1 nM et 10 µM, de préférence inférieures à 2 µM.

### Détermination de l'inhibition de la kinase Tie2 :

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.

L'effet inhibiteur des molécules est déterminé dans un test de phosphorylation de PLC par Tie2 en présence de GST-Tie2 purifiée à environ 80 % d'homogénéité. Le substrat est composé des fragments SH2-SH3 de la PLC exprimée sous forme de protéine de fusion GST.

L'activité kinase de Tie2 est mesurée dans un tampon MOPS 20mM pH 7,2, contenant 10 mM MgCl₂, 10 mM MnCl₂, 1 mM DTT, 10 mM de glycérophosphate. Dans une plaque 96 puits FlashPlate maintenue sur glace, on dépose un mélange réactionnel composé de 70 µl de tampon kinase contenant 100 ng d'enzyme GST-Tie2 par puits. Ensuite 10 µl de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi d'³³P[ATP]. Après 1 heure d'incubation à 37°C, la réacton est stoppée en ajoutant 1 volume (100 µl) d'EDTA à 200 mM. Après élimination du tampon d'incubation, les puits sont lavés trois fois avec 300 µl de PBS. La radioactivité est mesurée sur un MicroBeta1450 Wallac.

L'inhibition de l'activité Tie2 est calculée et exprimée en pourcentage d'inhibition par rapport à l'activité contrôle déterminée en l'absence de composé. Les composés inhibent la kinase Tie 2, à des concentrations généralement comprises entre 1 nM et 10 µM.

Les composés selon l'invention sont donc des inhibiteurs des kinases Aurora 1, Aurora 2 et Tie2. Par conséquent, ils peuvent être utilisés pour la préparation de médicaments, en particulier de médicaments inhibiteurs des kinases Aurora 1, Aurora 2 et Tie2 .

## Revendications

1. Composé de formule (I) : 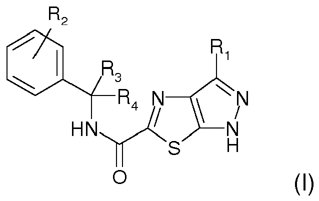 dans laquelle :
(i) R₁ représente un groupe -NHR₅, dans lequel R₅ est sélectionné parmi un atome d'hydrogène ou un groupe -COR₆, où R₆ est choisi parmi un groupe aryle ou hétéroaryle, éventuellement substitué par un atome d'halogène, -(C₁-C₃)alcoxy, -(C₀-C₃)alkyle-hétérocycloalkyle ;
(ii) R₂ représente un atome d'hydrogène ;
(iii) R₃ et R₄ représentent chacun un groupe méthyle ;
dans lequel :
- le groupe hétérocycloalkyle représente un groupe cycloalkyle comprenant de 3 à 8 atomes de carbone, comprenant en outre 1 à 4 hétéroatomes choisis parmi N, O ou S , ledit groupe hétérocycloalkyle étant choisi dans le groupe constitué de : azétidinyle, pyrrolidinyle, pipéridinyle, morpholinyle, pipérazinyle et N-alkyle-pipérazinyle ;
- le groupe alkyle représente un groupe hydrocarboné aliphatique saturé, linéaire ou ramifié comprenant de 1 à 20 atomes de carbone;
- le groupe aryle représente un groupe aromatique cyclique comprenant de 6 à 14 atomes de carbone ;
- le groupe hétéroaryle représente un groupe aromatique cyclique de 5 à 14 chaînons comprenant comme atome formant le cycle, un ou plusieurs hétéroatomes choisis parmi O, S ou N.

2. Composé selon la revendication 1 **caractérisé en ce que** le groupe aryle est un phényle et le groupe hétéroaryle est un thiényle ou une pyridine.

3. Composé selon la revendication 1 **caractérisé en ce que** l'hétérocycloalkyle est de la forme :

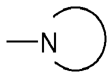

4. Composé selon l'une quelconque des revendications 1 à 3 **caractérisé en ce que** l'hétérocycloalkyle est choisi parmi morpholinyle, pipérazinyle, N-alkyle-pipérazinyle, pyrrolidinyle, pipéridinyle.

5. Composé selon la revendication 1 choisi dans la liste suivante :
• N-(1-méthyl-1-phényl-éthyl)-(3-amino-1H-pyrazolo[4,3-*d*]thiazole)-5-carboxamide ;
• N-(1-méthyl-1-phényl-éthyl)-3-[4-(4-méthyl-pipérazin-1-yl)-benzoylamino]-1H-pyrazolo[4,3-*d*]thiazole-5-carboxamide ;
• N-(1-méthyl-1-phényl-éthyl)-3-(4-morpholin-4-yl-benzoylamino)-1H-pyrazolo[4,3-*d*]thiazole-5-carboxamide ;
• N-(1-méthyl-1-phényl-éthyl)-3-(4-méthoxy-benzoylamino)-1-H-pyrazolo[4,3-*d*]thiazole-5-carboxamide ;
• N-(1-méthyl-1-phényl-éthyl)-3-[(thiophène-3-carbonyl)-amino]-1H-pyrazolo[4,3-*d*]thiazole-5-carboxamide ;
• N-(1-méthyl-1-phényl-éthyl)-3-(4-morpholin-4-ylméthyl-benzoylamino)-1H-pyrazolo[4,3-*d*]thiazole-5-carboxamide ;
• N-(1-méthyl-1-phényl-éthyl)-3-[4-(4-méthyl-piperazin-1-ylméthyl)-benzoylamino]-1H-pyrazolo[4,3-d]thiazole-5-carboxamide ;
• N-(1-méthyl-1-phényl-éthyl)-3-(4-pipéridin-1-ylméthyl-benzoylamino)-1H-pyrazolo[4,3-d]thiazole-5-carboxamide ;
• N-(1-méthyl-1-phényl-éthyl)-3-(4-pyrrolidin-1-ylméthyl-benzoylamino)-1H-pyrazolo[4,3-d]thiazole-5-carboxamide ;
• N-(1-méthyl-1-phényl-éthyl)-3-{[4-(4-méthyl-pipérazin-1-yl)-thiophène-2-carbonyl]-amino}-1H-pyrazolo[4,3-d]thiazole-5-carboxamide ;
• N-(1-méthyl-1-phényl-éthyl)-3-{[5-(4-méthyl-pipérazin-1-yl)-thiophène-2-carbonyl]-amino}-1H-pyrazolo[4,3-d]thiazole-5-carboxamide ;
• N-(1-méthyl-1-phényl-éthyl)-3-[(pyridine-2-carbonyl)-amino]-1H-pyrazolo[4,3-d]thiazole-5-carboxamide ;
• N-(1-méthyl-1-phényl-éthyl)-3-[(pyridine-3-carbonyl)-amino]-1H-pyrazolo[4,3-d]thiazole-5-carboxamide ;
• N-(1-méthyl-1-phényl-éthyl)-3-[(pyridine-4-carbonyl)-amino]-1H-pyrazolo[4,3-d]thiazole-5-carboxamide ;
• N-(1-méthyl-1-phényl-éthyl)-3-{[2-(4-méthyl-pipérazin-1-yl)-pyridine-4-carbonyl]-amino}-1H-pyrazolo[4,3-d]thiazole-5-carboxamide ;
• N-(1-méthyl-1-phényl-éthyl)-3-{[6-(4-méthyl-pipérazin-1-yl)-pyridine-2-carbonyl]-amino}-1H-pyrazolo[4,3-d]thiazole-5-carboxamide ;
• N-(1-méthyl-1-phényl-éthyl)-3-{[6-(4-méthyl-pipérazin-1-yl)-pyridine-3-carbonyl]-amino}-1H-pyrazolo[4,3-d]thiazole-5-carboxamide.

6. Composé selon l'une quelconque des revendications 1 à 5 sous forme d'hydrate ou de solvat et/ou de sel d'addition.

7. Médicament **caractérisé en ce qu'**il comprend un composé selon l'une quelconque des revendications 1 à 6.

8. Composition pharmaceutique comprenant un composé selon l'une quelconque des revendications 1 à 6 ainsi qu'au moins un excipient pharmaceutiquement acceptable.

9. Utilisation d'un composé selon l'une quelconque des revendications 1 à 6 pour la préparation d'un médicament destiné au traitement et à la prévention du cancer.

10. Procédé de préparation d'un composé de formule (Ib) suivante à partir d'un composé de formule (II) : 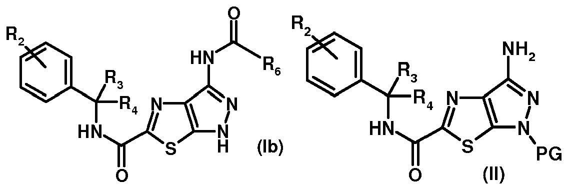 formules dans lesquelles R₂, R₃, R₄ et R₆ sont tels que définis à l'une quelconque des revendications 1 à 6 et PG désigne un groupement protecteur de la fonction NH du thiazole,
et comprenant dans l'ordre les étapes suivantes :
- l'acylation du composé de formule (II) avec un agent acylant permettant d'introduire R₆ (étape 2) ;
- la déprotection du composé issu de l'étape précédente (étape 3).

11. Procédé selon la revendication 10 dans lequel l'agent acylant est un chlorure d'acide R₆C(O)Cl, un anhydride (R₆CO)₂O ou un acide R₆C(O)H.

12. Procédé selon la revendication 10 ou 11 dans lequel l'étape 2 est précédée d'une étape de protection de la fonction NH du composé de formule (la) par le groupement protecteur PG :

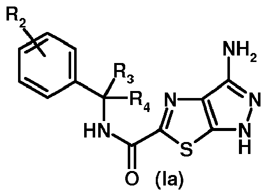

13. Procédé de préparation de composés (IIIb) à partir de composés (IIIa) comprenant l'étape suivante : 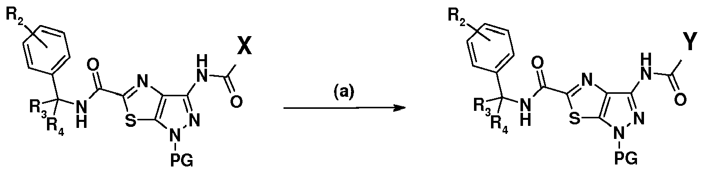 dans laquelle
**X** représente 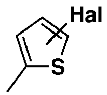 et **Y** représente 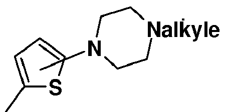 ou bien
**X** représente 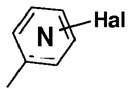 et **Y** représente 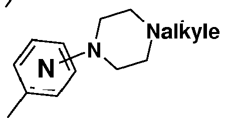 ou bien
**X** représente 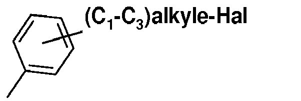 et **Y** représente 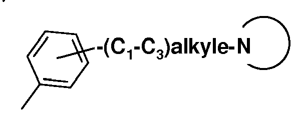
et dans laquelle R₂, R₃, sont tels que definis à l'une quelconque des revendications 1 à 6, et PG désigne un groupement protecteur de la fonction NH du thiazole.

14. Composé répondant à l'une des formules suivantes : 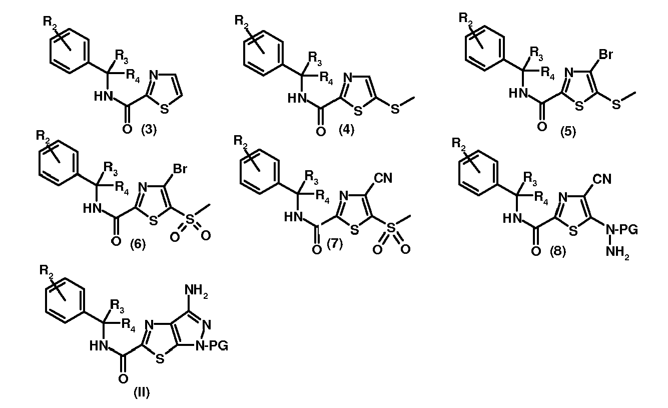 dans lesquelles R₂, R₃, R₄ sont tels que définis à l'une quelconque des revendications 1 à 6, et PG désigne un groupement protecteur de la fonction NH du thiazole

15. Composé répondant à l'une des formules suivantes : 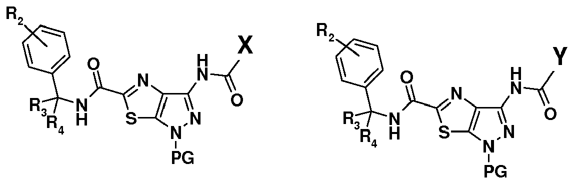 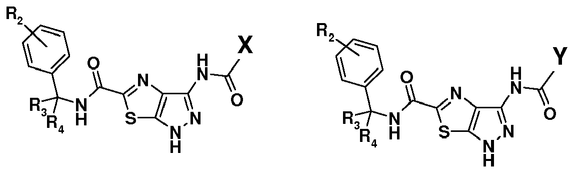
dans laquelle R₂, R₃, R₄ sont tels que définis à l'une quelconque des revendications 1 à 6, PG désigne un groupement protecteur de la fonction NH du thiazole, et
**X** représente 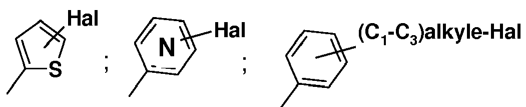
**Y** représente

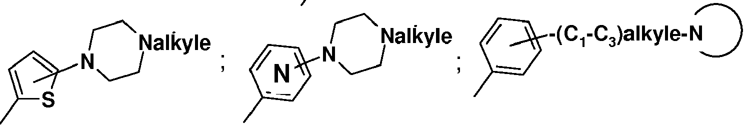

## Patentansprüche

1. Verbindung der Formel (I): 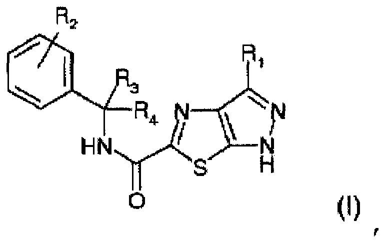 worin:
(i) R₁ eine Gruppe -NHR₅, worin R₅ aus einem Wasserstoffatom oder einer Gruppe -COR₆ ausgewählt ist, worin R₆ aus einer Arylgruppe oder Heteroarylgruppe, gegebenenfalls substituiert durch ein Halogenatom, -(C₁-C₃)-Alkoxy, -(C₀-C₃)-Alkylheterocycloalkyl ausgewählt ist, bedeutet;
(ii) R₂ ein Wasserstoffatom bedeutet;
(iii) R₃ und R₄ jeweils eine Methylgruppe bedeuten;
worin:
- die Heterocycloalkylgruppe eine Cycloalkylgruppe umfassend 3 bis 8 Kohlenstoffatome, weiterhin umfassend 1 bis 4 Heteroatome, ausgewählt aus N, O oder S, bedeutet, wobei die Heterocycloalkylgruppe aus der Gruppe bestehend aus: Azetidinyl, Pyrrolidinyl, Piperidinyl, Morpholinyl, Piperazinyl und N-Alkylpiperazinyl ausgewählt ist;
- die Alkylgruppe eine gesättigte, geradkettige oder verzweigte aliphatische Kohlenwasserstoffgruppe umfassend 1 bis 20 Kohlenstoffatome bedeutet;
- die Arylgruppe eine aromatische cyclische Gruppe umfassend 6 bis 14 Kohlenstoffatome bedeutet;
- die Heteroarylgruppe eine aromatische cyclische 5-bis 14-gliedrige Gruppe bedeutet, die als Atom, das den Ring bildet, ein oder mehrere Heteroatome, ausgewählt aus 0, S oder N, umfasst.

2. Verbindung nach Anspruch 1, **dadurch gekennzeichnet, dass** es sich bei der Arylgruppe um ein Phenyl und bei der Heteroarylgruppe um ein Thienyl oder ein Pyridin handelt.

3. Zusammensetzung nach Anspruch 1, **dadurch gekennzeichnet, dass** das Heterocycloalkyl die folgende Form aufweist:

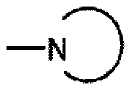

4. Verbindung nach einem der Ansprüche 1 bis 3, **dadurch gekennzeichnet, dass** das Heterocycloalkyl aus der Reihe Morpholinyl, Piperazinyl, N-Alkylpiperazinyl, Pyrrolidinyl, Piperidinyl ausgewählt ist.

5. Verbindung nach Anspruch 1, ausgewählt aus der folgenden Aufzählung:
• N-(1-Methyl-1-phenylethyl)-(3-amino-1H-pyrazolo[4,3-d]thiazol-5-carboxamid;
• N-(1-Methyl-1-phenylethyl)-3-[4-(4-methylpiperazin-1-yl)benzaylamino]-1H-pyrazolo[4,3-d]thiazol-5-carboxamid;
• N-(1-Methyl-1-phenylethyl)-3-(4-morpholin-4-ylbenzoylamino)-1H-pyrazolo[4,3-*d*]thiazol-5-carboxamid;
• N-(1-Methyl-1-phenylethyl)-3-(4-methoxybenzoylamino)-1H-pyrazolo[4,3-*d*]thiazol-5-carboxamid;
• N-(1-Methyl-1-phenylethyl)-3-[(thiophen-3-carbonyl)amino]-1H-pyrazolo[4,3-d]thiazol-5-carboxamid;
• N-(1-Methyl-1-phenylethyl)-3-(4-morpholin-4-ylmethylbenzoylamino)-1H-pyrazolo[4,3-*d*]thiazol-5-carboxamid;
• N-(1-(Methyl-1-phenylethyl)-3-[4-(4-methylpiperazin-1-ylmethyl)benzoylamino]-1H-pyrazolo[4,3-d]thiazol-5-carboxamid;
• N-(1-Methyl-1-phenylethyl)-3-(4-piperidin-1-ylmethylbenzoylamino)-1H-pyrazolo[4,3-d]thiazol-5-carboxamid;
• N-(1-Methyl-1-phenylethyl)-3-{4-pyrrolidin-1-ylmethylbenzoylamino)-1H-pyrazolo[4,3-*d*]thiazol-5-carboxamid;
• N-(1-Methyl-1-phenylethyl)-3-{[4-(4-methylpiperazin-1-yl)thiophen-2-carbonyl]amino}-1H-pyrazolo[4,3-*d*]thiazol-5-carboxamid;
• N-(1-Methyl-1-phenylethyl)-3-{[5-(4-methylpiperazin-1-yl)thiophen-2-carbonyl]amino}-1H-pyrazolo[4,3-d]thiazol-5-carboxamid;
• N-(1-Methyl-1-phenylethyl)-3-[(pyridin-2-carbonyl)amino]-1H-pyrazolo[4,3-d]thiazol-5-carboxamid;
• N-(1-Methyl-1-phenylethyl)-3-[(pyridin-3-carbonyl)amino]-1H-pyrazolo[4,3-d]thiazol-5-carboxamid;
• N-(1-Methyl-1-phenylethyl)-3[(pyridin-4-carbonyl)amino]-1H-pyrazolo[4,3-d]thiazol-5-carboxamid;
• N-(1-Methyl-1-phenylethyl)-3{[2-(4-methylpiperazin-1-yl)pyridin-4-carbonyl]amino}-1H-pyrazolo[4,3-d]thiazol-5-carboxamid;
• N-(1-methyl-1-phenylethyl)-3-{[6-(4-methylpiperazin-1-yl)pyridin-2-carbonyl]amino}-1H-pyrazolo[4,3-d]thiazol-5-carboxamid;
• N-(1-Methyl-1-phenylethyl)-3-{[6-(4-methylpiperazin-1-yl)pyridin-3-carbonyl]amino}-1H-pyrazolo[4,3-d]thiazol-5-carboxamid.

6. Verbindung nach einem der Ansprüche 1 bis 5 in Form des Hydrats oder Solvats und/oder des Säureadditionssalzes.

7. Arzneimittel, **dadurch gekennzeichnet, dass** es eine Verbindung nach einem der Ansprüche 1 bis 6 umfasst.

8. Pharmazeutische Zusammensetzung, die eine Verbindung nach einem der Ansprüche 1 bis 6 sowie mindestens einen pharmazeutisch unbedenklichen Grundstoff umfasst.

9. Verwendung einer Verbindung nach einem der Ansprüche 1 bis 6 für die Herstellung eines Arzneimittels zur Behandlung und Vorbeugung von Krebs.

10. Verfahren zur Herstellung einer Verbindung der folgenden Formel (Ib), ausgehend von einer Verbindung der Formel (II): 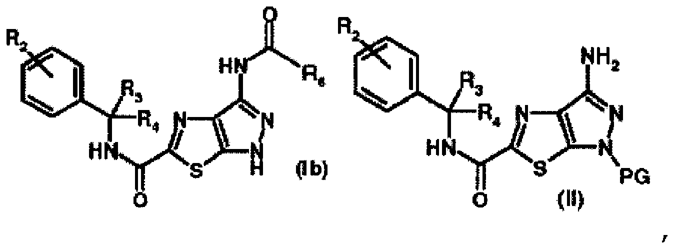 wobei in diesen Formeln R₂, R₃, R₄ und R₆ wie in einem der Ansprüche 1 bis 6 definiert sind und PG eine Schutzgruppe der NH-Funktion des Thiazols bedeutet,
und umfassend die folgenden Schritte in der folgenden Reihenfolge:
- Acylierung der Verbindung der Formel (II) mit einem Acylierungsmittel, das es gestattet, R₆ einzuführen (Schritt 2);
- Entschützen der in dem vorhergehenden Schritt erhaltenen Verbindung (Schritt 3).

11. Verfahren nach Anspruch 10, wobei es sich bei dem Acylierungsmittel um ein Säurechlorid R₆C(O)Cl, ein Anhydrid (R₆CO)₂O oder eine Säure R₆C(O)H handelt.

12. Verfahren nach Anspruch 10 oder 11, wobei vor Schritt 2 ein Schritt des Schützens der NH-Funktion der Verbindung der Formel (Ia) durch die Schutzgruppe PG erfolgt:

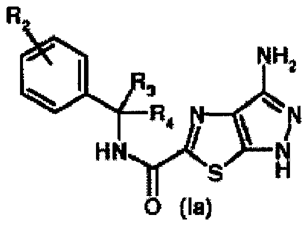

13. Verfahren zur Herstellung von Verbindungen (IIIb) ausgehend von Verbindungen (IIIa), umfassend den folgenden Schritt: 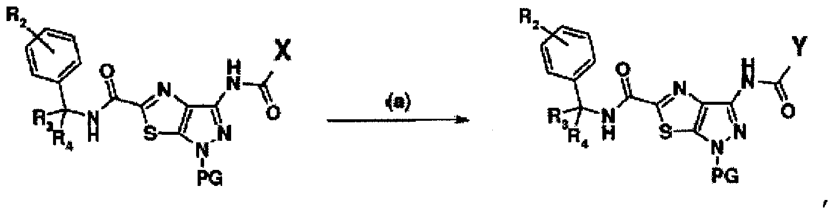 worin
**X** 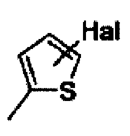 bedeutet und **Y** 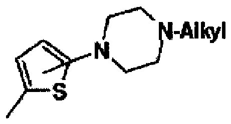 oder **X** 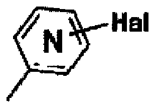 bedeutet und **Y** 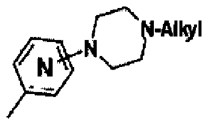 oder **X** 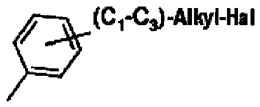 und **Y** 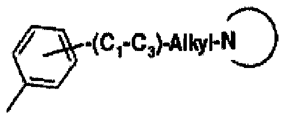 und worin R₂, R₃ wie in einem der Ansprüche 1 bis 6 definiert sind und PG eine Schutzgruppe der NH-Funktion des Thiazols bedeutet.

14. Verbindung, die einer der folgenden Formeln entspricht: 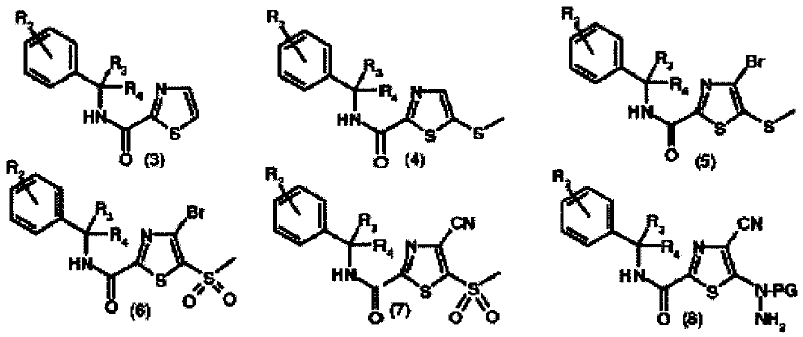 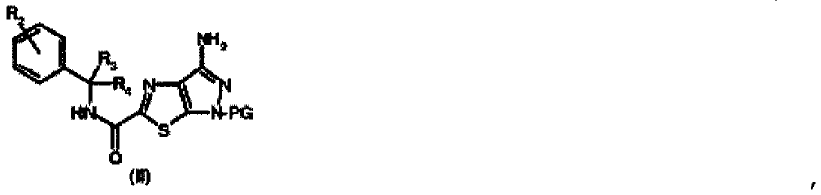 worin R₂, R₃, R₄ wie in einem der Ansprüche 1 bis 6 definiert sind und PG eine Schutzgruppe der NH-Funktion des Thiazols bedeutet.

15. Verbindung, die einer der folgenden Formeln entspricht: 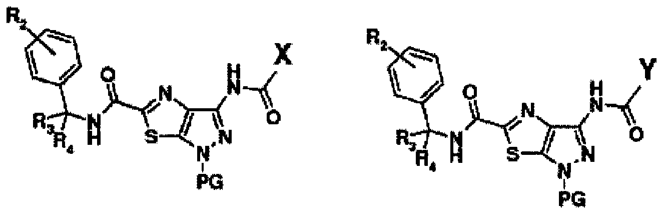 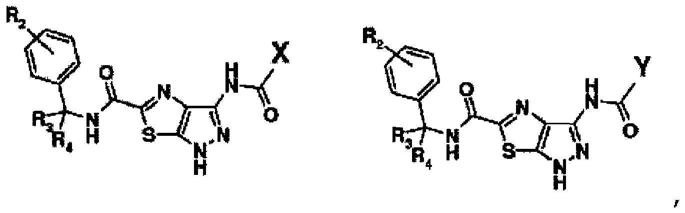 worin R₂, R₃, R₄ wie in einem der Ansprüche 1 bis 6 definiert sind und PG eine Schutzgruppe der NH-Funktion des Thiazols bedeutet und
**X** 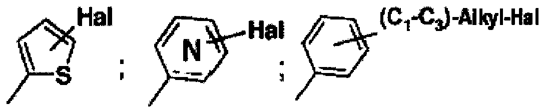 bedeutet
**Y** 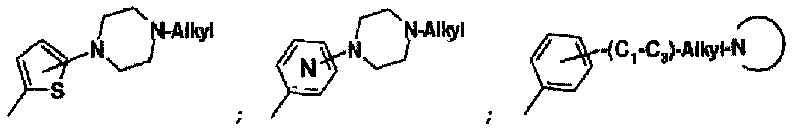 bedeutet.

## Claims

1. Compound of formula (I): 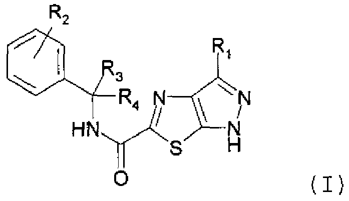 in which:
(i) R₁ represents a group -NHR₅, in which R₅ is selected from a hydrogen atom and a group -COR₆, in which R₆ is chosen from an aryl or heteroaryl group, optionally substituted with a halogen atom, -(C₁-C₃)alkoxy or -C₀-C₃)alkyl-heterocycloalkyl;
(ii) R₂ represents a hydrogen atom;
(iii) R₃ and R₄ each represent a methyl group; in which:
- the heterocycloalkyl group represents a cycloalkyl group comprising from 3 to 8 carbon atoms, also comprising 1 to 4 heteroatoms chosen from N, O and S, the said heterocycloalkyl group being chosen from the group consisting of: azetidinyl, pyrrolidinyl, piperidinyl, morpholinyl, piperazinyl and N-alkylpiperazinyl;
- the alkyl group represents a linear or branched, saturated aliphatic hydrocarbon-based group comprising from 1 to 20 carbon atoms;
- the aryl group represents a cyclic aromatic group comprising from 6 to 14 carbon atoms;
- the heteroaryl group represents a 5- to 14-membered cyclic aromatic group comprising, as atom forming the ring, one or more heteroatoms chosen from O, S and N.

2. Compound according to Claim 1, **characterized in that** the aryl group is a phenyl and the heteroaryl group is a thienyl or a pyridine.

3. Compound according to Claim 1, **characterized in that** the heterocycloalkyl is of the form:

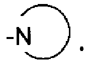

4. Compound according to any one of Claims 1 to 3, **characterized in that** the heterocycloalkyl is chosen from morpholinyl, piperazinyl, N-alkylpiperazinyl, pyrrolidinyl and piperidyl.

5. Compound according to Claim 1, chosen from the following list:
• N-(1-methyl-1-phenylethyl)(3-amino-1H-pyrazolo[4,3-d]thiazole)-5-carboxamide;
• N-(1-methyl-1-phenylethyl)-3-[4-(4-methylpiperazin-1-yl)benzoylamino]-1H-pyrazolo[4,3-d]thiazole-5-carboxamide;
• N-(1-methyl-1-phenylethyl)-3-(4-morpholin-4-ylbenzoylamino)-1H-pyrazolo[4,3-d]thiazole-5-carboxamide;
• N-(1-methyl-1-phenylethyl)-3-(4-methoxybenzoylamino)-1-H-pyrazolo[4,3-d]thiazole-5-carboxamide;
• N-(1-methyl-1-phenylethyl)-3-[(thiophene-3-carbonyl)amino]-1H-pyrazolo[4,3-*d*]thiazole-5-carboxamide;
• N-(1-methyl-1-phenylethyl)-3-(4-morpholin-4-ylmethylbenzoylamino)-1H-pyrazolo[4,3-d]thiazole-5-carboxamide;
• N-(1-methyl-1-phenylethyl)-3-[4-(4-methylpiperazin-1-ylmethyl)benzoylamino]-1H-pyrazolo[4,3-d]thiazole-5-carboxamide;
• N-(1-methyl-1-phenylethyl)-3-(4-piperidin-1-ylmethylbenzoylamino)-1H-pyrazolo[4,3-d]thiazole-5-carboxamide;
• N-(1-methyl-1-phenylethyl)-3-(4-pyrrolidin-1-ylmethylbenzoylamino)-1H-pyrazolo[4,3-d]thiazole-5-carboxamide;
• N-(1-methyl-1-phenylethyl)-3-{[4-(4-methyl-piperazin-1-yl)thiophene-2-carbonyl]amino}-1H-pyrazolo[4,3-d]thiazole-5-carboxamide;
• N-(1-methyl-1-phenylethyl)-3-{[5-(4-methylpiperazin-1-yl)thiophene-2-carbonyl]amino}-1H-pyrazolo[4,3-d]thiazole-5-carboxamide;
• N-(1-methyl-1-phenylethyl)-3-[(pyridine-2-carbonyl)amino]-1H-pyrazolo[4,3-d]thiazole-5-carboxamide;
• N-(1-methyl-1-phenylethyl)-3-[(pyridine-3-carbonyl)amino]-1H-pyrazolo[4,3-d]thiazole-5-carboxamide;
• N-(1-methyl-1-phenylethyl)-3-[(pyridine-4-carbonyl)amino]-1H-pyrazolo[4,3-d]thiazole-5-carboxamide;
• N-(1-methyl-1-phenylethyl)-3-{[2-(4-methylpiperazin-1-yl)pyridine-4-carbonyl]amino}-1H-pyrazolo[4,3-d]thiazole-5-carboxamide;
• N-(1-methyl-1-phenylethyl)-3-{[6-(4-methylpiperazin-1-yl)pyridine-2-carbonyl]amino}-1H-pyrazolo[4,3-d]thiazole-5-carboxamide;
• N-(1-methyl-1-phenylethyl)-3-{[6-(4-methylpiperazin-1-yl)pyridine-3-carbonyl]amino}-1H-pyrazolo[4,3-d]thiazole-5-carboxamide.

6. Compound according to any one of Claims 1 to 5, in hydrate, solvate and/or addition-salt form.

7. Medicament, **characterized in that** it comprises a compound according to any one of Claims 1 to 6.

8. Pharmaceutical composition comprising a compound according to any one of Claims 1 to 6 and also at least one pharmaceutically acceptable excipient.

9. Use of a compound according to any one of Claims 1 to 6 for the preparation of a medicament for treating and preventing cancer.

10. Process for preparing a compound of formula (Ib) below from a compound of formula (II): 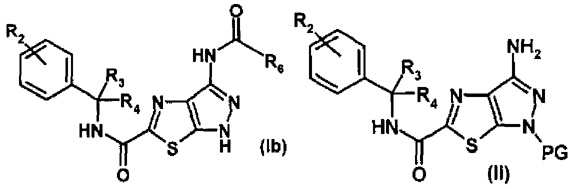 in which formulae R₂, R₃, R₄ and R₆ are as defined in any one of Claims 1 to 6 and PG denotes a protecting group for the NH function of the thiazole,
and comprising, in order, the following steps:
- acylation of the compound of formula (II) with an acylating agent, allowing the introduction of R₆ (step 2);
- deprotection of the compound obtained from the preceding step (step 3),

11. Process according to Claim 10, in which the acylating agent is an acid chloride R₆C(O)Cl, an anhydride (R₆CO)₂O or an acid R₆C(O)H.

12. Process according to Claim 10 or 11, in which step 2 is preceded by a step of protecting the NH function of the compound of formula (Ia) with the protecting group PG:

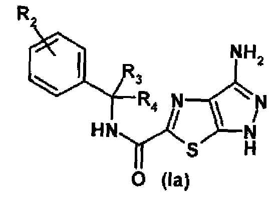

13. Process for preparing the compounds (IIIb) from the compounds (IIIa), comprising the following step: 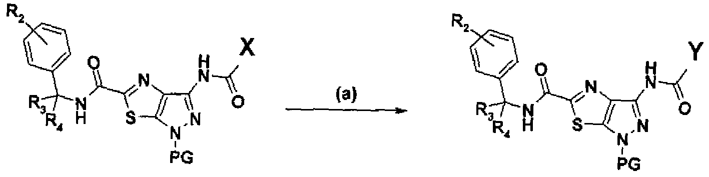 in which:
**X** represents 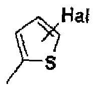 and **Y** represents 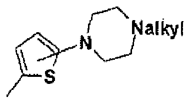 or
**X** represents 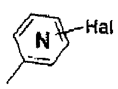 and **Y** represents 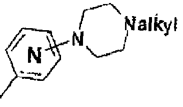 or alternatively
**X** represents 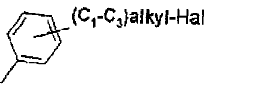 and **Y** represents 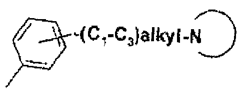 and in which R₂ and R₃ are as defined in any one of Claims 1 to 6 and PG denotes a protecting group for the NH function of the thiazole.

14. Compound corresponding to one of the following formulae: 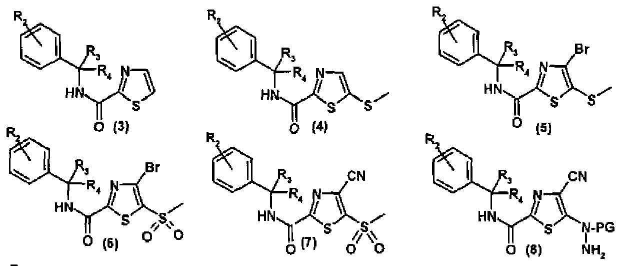 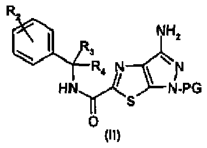 in which R₂, R₃ and R₄ are as defined in any one of Claims 1 to 6 and PG denotes a protecting group for the NH function of the thiazole.

15. Compound corresponding to one of the following formulae: 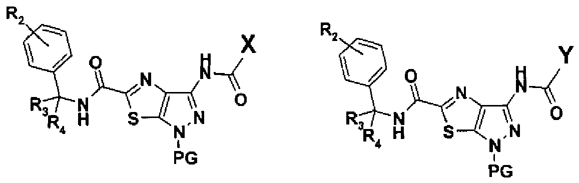 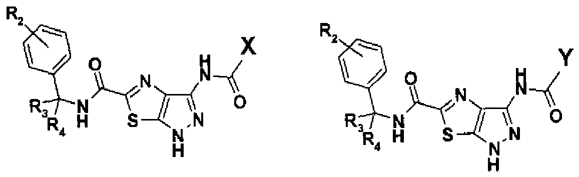 in which R₂, R₃ and R₄ are as defined in any one of Claims 1 to 6, PG denotes a protecting group for the NH function of the thiazole and
**X** represents 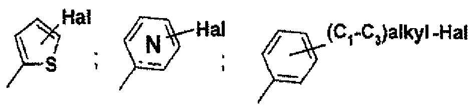 **Y** represents
